# EUROPEAN PATENT SPECIFICATION

(11) **EP 3 784 317 B1**
(45) Date of publication and mention of the grant of the patent: **18.03.2026**
(21) Application number: 19791709.9
(22) Date of filing: 24.04.2019
(51) Int. Cl.: A61M 15/00, A61M 16/00, A61B 5/087

(54) **ACCESSORY APPARATUS FOR AN INHALER**
ZUBEHÖRGERÄT FÜR EIN INHALATOR
APPAREIL ACCESSOIRE POUR INHALATEUR

(30) Priority: 24.04.2018 US 201862662051 P
(43) Date of publication of application: 03.03.2021
(73) Proprietor: MannKind Corporation, Westlake Village, CA 91362 (US)
(72) Inventor: POCREVA III, John, J., Lagrangeville, NY 12540 (US); ADAMO, Benoit, Salem, NY 10590 (US); LAURENZI, Brendan, Middlebury, CA 06762 (US); SMUTNEY, Chad, C., Watertown, CT 06795 (US); KINSEY, Spencer, P., Sandy Hook, CT 06482 (US)
(74) Representative: AWA Sweden AB
(86) International application number: PCT/US2019/028986
(87) International publication number: WO 2019/209994

(56) References cited:
- EP-A2- 2 609 954
- WO-A1-2015/144442
- WO-A1-2016/033418
- WO-A1-2017/178865
- WO-A1-2017/201463
- DE-A1- 102008 051 515
- US-A1- 2004 241 232
- US-B1- 6 328 033

## Description

### CROSS REFERENCE TO RELATED APPLICATIONS

This application claims the benefit of Provisional Patent Application Serial No. 62/662,051 filed April 24, 2018.

### TECHNICAL FIELD

Described herein are interactive apparatuses and methods for recording, transferring and displaying physical measurements based on physiological conditions generated by an inhaler and/or a subject during an inhalation maneuver in real-time.

### BACKGROUND

Inhaler devices for dispensing therapeutic substances via the respiratory tract, in particular, for pulmonary delivery in treating local or systemic diseases are commercially available. For example, nebulizers, devices containing propellants, and dry powder inhalers have been used for the treatment of diseases, such as asthma, respiratory tract infections and systemic diseases such as diabetes.

The efficiency of delivering the required dosage of a therapeutic substance to a patient in treating a disease depends on the efficiency of the device, and overall efficiency can be enhanced by providing proper feedback mechanisms to a patient, clinician or physician during use of the device to teach, for example, proper inhalation techniques to a patient. Improper use of the device and poor inhalation technique can lead to lack of efficacy in treating a disease. For example, administering lower or higher dosages of a therapeutic substance than intended can be harmful to a patient. To effectively deliver therapeutic substances to the respiratory tract, a patient or user can be trained or coached to use the device in an appropriate manner.

Dry powder inhalers used to deliver medicaments to the lungs contain a dose of a powder formulation usually either in bulk supply or quantified into individual doses stored in unit dose compartments, like hard gelatin capsules, cartridges, or blister packs. Dosing reproducibility requires that the drug formulation is uniform and that the dose can be delivered to the patient with consistent and reproducible results. Therefore, dosing can be improved by optimizing discharge of a formulation, which is effectuated, for example, by having patients perform proper inhalation maneuvers that achieve the necessary dosing.

Devices for training patients to properly deliver therapeutic substances by the pulmonary tract are described, for example, in U.S. Patent No. 5,333,106, which discloses an apparatus for interactive training of a patient in use of an aerosol inhaler, including a feedback display based upon air flow versus volume data using a proper sequence of inhalation steps. U.S. Patent Application No. 10/759,859 (Publication No. US 2004/0187869) discloses a training device for medicament inhalers, for example, dry powder inhalers, which is based on measuring pressure differential and displaying a single value corresponding to both inhalation rapidity and inhalation flow rate peak, using a dry powder inhaler simulator.

Dry powder inhalers and cartridge systems, such as those described in U.S. Patents Nos. 8,499,757 and 8,636,001 can generate primary drug particles or suitable inhalation plumes during an inspiratory maneuver by deagglomerating a powder formulation within the inhaler and capsule or cartridge. The benefits of delivering drugs via pulmonary circulation are numerous and, include rapid entry into arterial circulation, avoidance of first pass drug degradation by liver metabolism and ease of use, for example, the lack of discomfort compared to other routes of administration such as by injection. These devices have been in use in clinical settings and are now commercially available.

An interactive apparatus and method for profiling of inhalation efforts is disclosed in U.S. Patent 9,364,619, patent publication WO2017/201463 shows a prior art inhaler accessory apparatus.

There is a need in the art for improvements in design and manufacture of inhaler devices which would maximize accuracy and require minimal training and effort for subjects in proper use of the inhalation system and monitoring patients during use of the inhalation system and their overall course of care and improvements to the flexibility of application to inhalers and inhaler parts including medicament packages and the reusability of such systems overall. The present disclosure presents apparatus and methods that achieve these goals.

### SUMMARY

The invention is in accordance with the appended claims.

### BRIEF DESCRIPTION OF THE DRAWINGS

FIG. 1 illustrates an isometric view of an embodiment of a wireless dry powder detecting and sensing inhaler accessory apparatus mounted on an inhaler.
FIG. 2 illustrates an isometric view of an embodiment of a wired dry powder detecting and sensing inhaler accessory apparatus embodiment mounted on an inhaler.
FIG. 3 illustrates a top isometric view of an embodiment of a wireless detecting and sensing inhaler accessory apparatus.
FIG. 4 illustrates a bottom isometric view of an embodiment of a wireless detecting and sensing inhaler accessory apparatus, showing an electronic board.
FIG. 5 illustrates a top view of the electronic board of FIG. 4.
FIG. 6 illustrates an isometric view of a dry powder inhaler coupled to an embodiment of a detecting and sensing inhaler accessory apparatus as shown in FIG. 1 including integrated signal indicating buttons.
FIG. 7 illustrates an isometric view of a dry powder inhaler coupled to an embodiment of a detecting and sensing inhaler accessory apparatus as shown in FIG. 2 including local signal indicating buttons.
FIG. 8 illustrates an isometric view of an embodiment of a wireless dry powder detecting and sensing inhaler accessory apparatus mounted on an inhaler and including an integrated display screen.
FIG. 9 illustrates a block diagram of an overall embodiment of a wireless detection and monitoring system disclosed herein.
FIG. 10 illustrates a block diagram of an embodiment of a detection and monitoring system disclosed herein.
FIG. 11 illustrates a block diagram of another embodiment of a detection and monitoring system disclosed herein.
FIG. 12 graphically illustrates an inhalation maneuver performed by a subject coached to take a breath for the purposes of monitoring inhalation effectiveness for dosing.
FIG. 13 illustrates a block diagram of an embodiment of a wireless detection and monitoring system disclosed herein where the inhaler accessory apparatus includes pressure sensors.
FIG. 14 illustrates a block diagram of an embodiment of a detection and monitoring system disclosed herein where the inhaler accessory apparatus includes pressure sensors and a display.
FIG. 15 illustrates a block diagram of another embodiment of a detection and monitoring system disclosed herein where the inhaler accessory apparatus includes pressure sensors and visual indicators.
FIG. 16 illustrates a block diagram of an embodiment of a wireless detection and monitoring system disclosed herein where the inhaler accessory apparatus includes a color detection sensor and pressure sensors.
FIG. 17 illustrates a block diagram of an embodiment of a detection and monitoring system disclosed herein where the inhaler accessory apparatus includes a color detection sensor and a display.
FIG. 18 illustrates a block diagram of another embodiment of a detection and monitoring system disclosed herein where the inhaler accessory apparatus includes a color detection sensor and visual indicators.
FIG. 19 illustrates a method of training or monitoring inhalation in a user with the system of FIG. 16.
FIG. 20 illustrates an isometric view of an embodiment of a wireless dry powder detecting and sensing inhaler accessory apparatus.
FIG. 21 illustrates an isometric, bottom view of an embodiment of a wireless dry powder detecting and sensing inhaler accessory apparatus embodiment of FIG. 20.
FIG. 22 illustrates a top isometric view of an embodiment of a wireless detecting and sensing inhaler accessory apparatus of FIG. 20 mounted on an inhaler embodiment.
FIG. 23 is a screen shot of a mobile phone display which graphically illustrates a standard baseline curve for use to measure and monitor inhalation efforts of subjects during training to use a dry powder inhaler.
FIG. 24 graphically illustrates an inhalation maneuver performed by a subject coached to take a breath for the purposes of monitoring inhalation effectiveness for dosing, wherein the resultant inhalation efforts is satisfactory.
FIG. 25 graphically illustrates an inhalation maneuver performed by a naive subject to take a breath for the purposes of monitoring inhalation effectiveness for dosing, wherein the resultant inhalation effort failed the parameters required to use the inhaler properly.
FIG. 26 graphically illustrates an inhalation maneuver performed by a naïve subject to take a breath for the purposes of monitoring inhalation effectiveness for dosing, wherein the resultant inhalation effort failed the parameters required to use the inhaler properly.
FIG. 27 illustrates a method of training or monitoring inhalation in a user with the system of FIG. 13.
FIG. 28 illustrates a method of training or monitoring inhalation in a user with the system of FIG. 13.

### DETAILED DESCRIPTION

Disclosed herein are apparatus and/or devices with interactive system and methods for measuring or monitoring real-time characteristic changes in pressure or pressure drop and/or flow from a subject during an inhalation maneuver with an inhaler. The devices can be used for detecting and monitoring and consequently training a subject to maximize efficiency of their respiratory maneuvers in conjunction with an inhalation device, and can also be used for monitoring inspiration during delivery of a medicament, to detect proper dose delivery, timing of dose delivery and proper performance of the inhalation system in use. In one example embodiment, the sensing and monitoring apparatus can be applied in conjunction with a high resistance inhaler. In embodiments herein, the detection and monitoring system can measure many characteristic parameters of an inhalation maneuver using inhalers, in particular, in conjunction with dry powder inhalers, which include data generated for assessing peak inhalation effort within two seconds of onset of an inhalation (PIP₂), total inhalation effort in the first second of an inhalation (AUC₁), total inhaled volume and the duration of an inhalation of patient inhalation efforts. Although the handheld inhaler system is described as comprising two parts - an inhaler and an inhaler accessory apparatus, one skilled in the art can appreciate that the inventive design of this system and method for measuring or monitoring data and characteristics during an inhalation maneuver can also apply to a device where the accessory features are integrated into the inhaler itself, albeit sacrificing flexibility and reusability.

The apparatus comprises an inhaler accessory apparatus adapted for mounting on or otherwise associating with an inhaler. The apparatus comprises at least one transducer or sensor which can detect at least one measurement, including pressure, air flow, air volume, humidity, and temperature, and convert such into an electrical signal. In some embodiments, the sensor can comprise a Doppler sensing device which can detect a flow of air or a gas through an inhaler. In other embodiments, the sensor comprises a pressure sensor which can detect pressure drop during an inhalation maneuver. The inhaler accessory apparatus can further include an electronic board with circuit elements including appropriate signal conditioning circuitry, such as signal filtering, amplification and analog to digital conversion, and processing circuitry such as a microprocessor, wired or wireless communication interface and the like to transfer the generated signal concurrently or in real-time to a receiving computer or personal data assistant (PDA), including a mobile telephone for display of the signal or processed information. In some embodiments, the output display can be an interactive display so that the display device provides a visual aid for allowing a physician and/or patient to view the inhalation maneuver parameters attained. In this manner, the information obtained can serve as a teaching guide for a subject to perform repeatable inhalation maneuvers in real-time, thereby facilitating proper inhalation delivery of a medicament when is self- administered. In another example embodiment, the data can be stored to be analyzed at a later date.

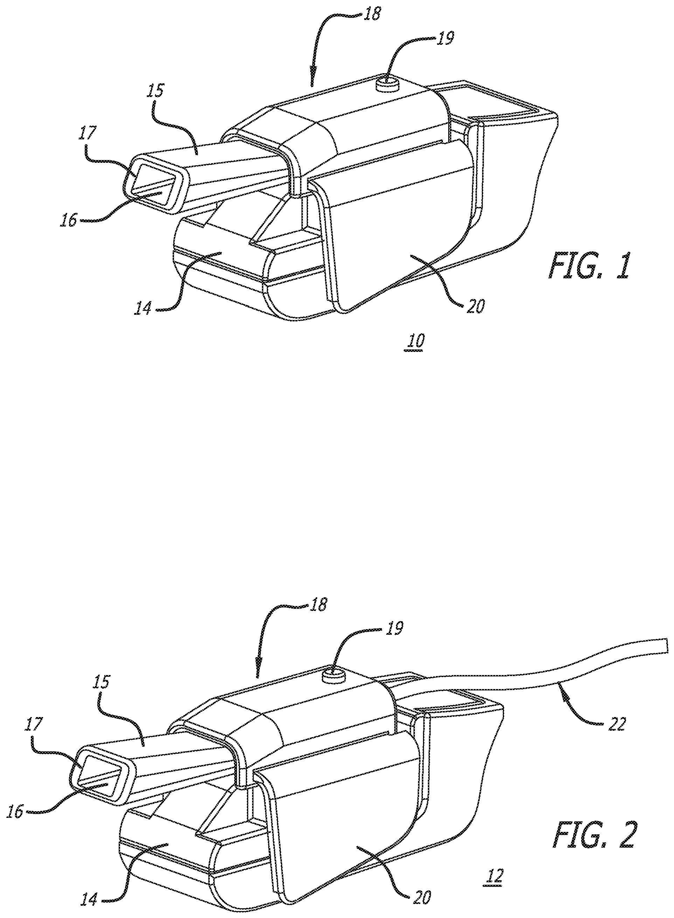

FIGs. 1 through 7 illustrate embodiments of a dry powder inhaler system or training device and its component parts. The training devices interactive systems described herein have been adapted to a high resistance dry powder inhaler as disclosed in U.S. Patents Nos. 8499,757, U.S. Patent No. 8,636,001 and U.S. Provisional Patent Application Serial No. 62/289,095.

FIG. 1 and FIG 2 depict, respectively, a wireless and wired inhalation detection and monitoring system **10, 12.** The system comprises an inhaler **14** comprising a mouthpiece **15** having an air conduit **16** and an air outlet port **17** for delivering a powder to a user/patient. The inhalation detection and monitoring system **10, 12** also comprises an inhaler accessory apparatus **18** adapted for mounting on, connecting with or otherwise associating with the inhaler **14.** In this embodiment, the inhaler accessory apparatus includes an actuator button **19** for powering ON/OFF the system **10, 12.** An air conduit is established between one or more air inlet ports for establishing air conduit pathways through the system which at least one air conduit pathway travels through a receptacle containing a dry powder for delivery to an individual in use. In some embodiments, the inhaler does not contain any powder during training of a patient for proper use of the inhaler. In the embodiment of FIGs. 1 and 2, inhaler **14** is of the same type, which is a dry powder inhaler, and the inhaler accessory apparatus **18** is adaptable to the top surface of inhaler **14.** FIG. 2 depicts inhaler accessory apparatus **18** having a wire **22** connected to the system for connecting to a power source and/or to a computer.

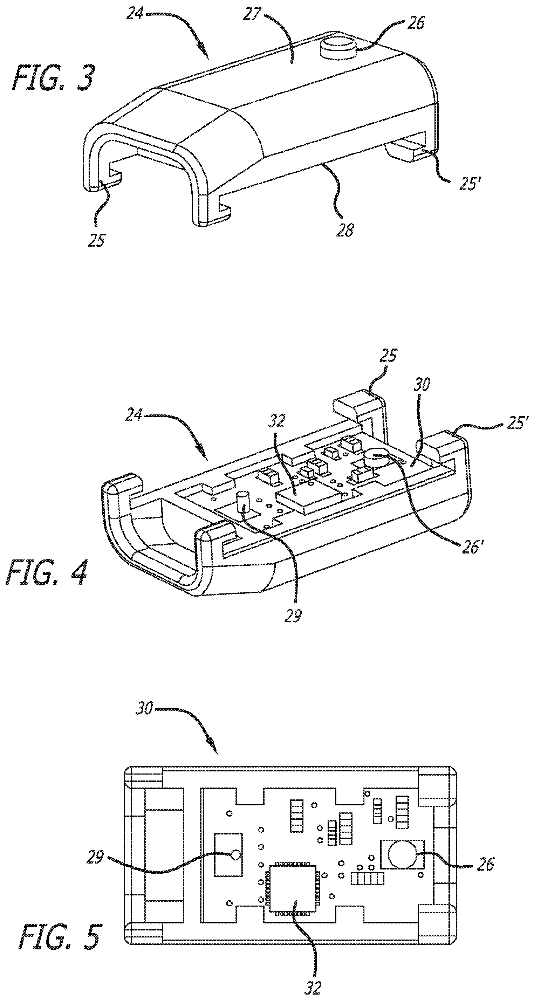

FIG. 3 illustrates a top isometric view of another embodiment of an inhaler accessory apparatus **24** designed for adapting to an inhaler. FIG. 4 illustrates a bottom isometric view of the apparatus **24.** As can be seen in FIGs. 3 and 4, accessory apparatus **24** preferably comprises a body having tabs **25, 25'** to attach to an inhaler. However, other types of securing devices known to those skilled in the art can be used to engage apparatus with inhaler. The apparatus **24** also preferably includes an actuator button **26** for activating the apparatus for use. In this embodiment, the body has a top surface **27,** a bottom surface **28** and an electronic board 30 mounted to the bottom surface. FIG. 4 illustrates an embodiment of an inhaler accessory apparatus **24** having an electronic board **30** integrally built into its undersurface **28.** FIG. 4 and FIG. 5 further illustrate electronic board **30.** Electronic board **30** preferably comprises actuator **26'** which is mechanically or otherwise connected to actuator button **26,** sensor **29,** and microprocessor **32.** Microprocessor **32** provides for actuating, detecting, processing signals from an associated inhaler and communicating the information/signals to a display device. In this embodiment, electronic board **30** is configured as a signal processing/interface board. Sensor **29** can be any type of sensor such as an acoustic sensor for detecting sound generated during an inhalation or a pressure sensor for detecting pressure drops during an inhalation. The inhaler accessory apparatus **24** is also preferably provided with a battery as a power source for activating the system when the actuator button is depressed. One skilled in the art will appreciate that the electronics included in inhaler accessory apparatus **24** can be provided as separate circuit components on separate boards connected by appropriate means as necessary for functionality. For instance, the microprocessor **32** can reside on a separate board from sensor **29** due to necessity of placement of the sensor **29.**

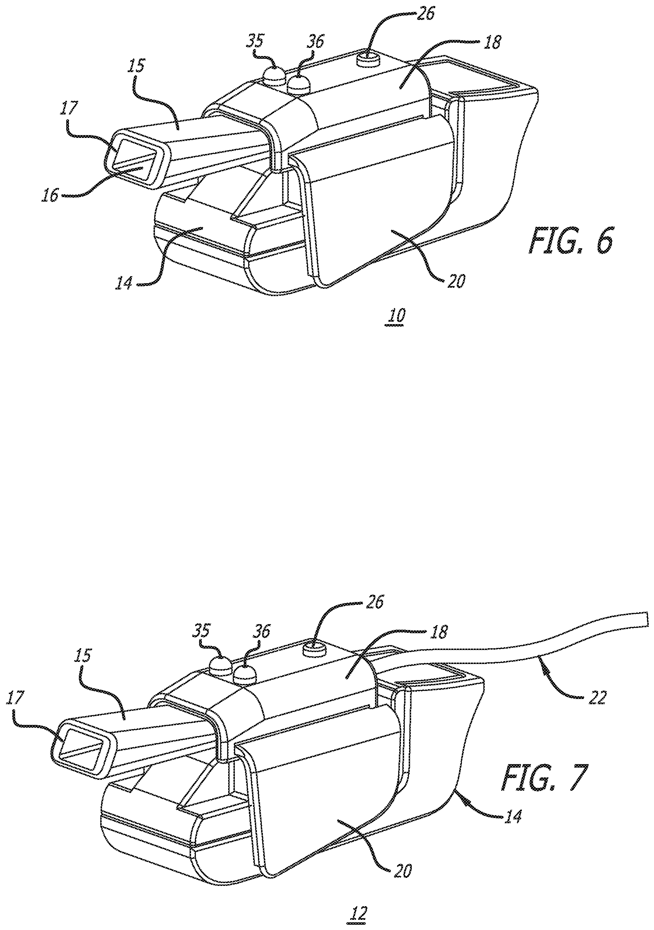

In another embodiment, the inhalation detection and monitoring system is provided with indicators as shown in FIGs. 6 and 7. FIG. 6 illustrates an isometric view of a dry powder inhaler coupled to an inhaler accessory apparatus as shown in FIG. 1 showing signal indicators **35,** 36. FIG. 7 illustrates an isometric view of a dry powder inhaler coupled to an inhaler accessory apparatus **18** as shown in FIG. 2 showing signal indicators **35,** 36. Signal indicators **35, 36** are preferably light emitting diodes or other light indicators for indicating certain status to the user. For example, they can be used to indicate whether an inhalation resulted in successful inhalation of the medicament. In this case, for example, one indicator can show a red signal light and another can show a green signal light during operation. The signal indicators **35, 36** would correspondingly indicate fail or pass to a user regarding effective delivery of a powder in an inhaler for treatment of a disease. A fail inhalation indicator (red light) indicates that the subject or patient's inhalation maneuver executed did not meet one or more predetermined criteria for inhaling a powder dose contained in the inhaler, and a pass inhalation indicator (green light) indicates that the subject or patient's inhalation maneuver meets the appropriate criteria for delivering a powder dose contained in the inhaler. Alternatively, only one signal indicator can be used if color can be selected based upon status or, for instance, if flashing can be used to indicate status. Signal indicators **35, 36** can be position anywhere within the jacket to facilitate visual perception by a user.

In another embodiment, an LED signal can also be integrated into the inhalation accessory apparatus and used to indicate the quality of an inhalation maneuver. For example, a solid red light indicator may indicate to a user that an inhalation has failed and in subsequent tries, the inhalation must be performed harder, deeper or faster. In one embodiment, a blinking red light indicator can signal to a user that the inhalation has failed and that a subsequent inhalation must be performed for a longer period of time. In this and other embodiments, a solid green light indicator denotes the inhalation passed or was acceptable to deliver the contents of a dry powder inhaler to a subject during an inhalation. Other uses of signal indicators **35, 36** could include power ON/OFF, power failure or battery low indication or status of connection between accessory apparatus and inhaler.

In an alternate embodiment, the inhalation detection and monitoring system is provided with indicators, including an annunciator to report on the quality of the inhalation maneuver. In this embodiment, the annunciator can be optionally provided to be activated separately, and it is particularly suitable for user with visual impairment.

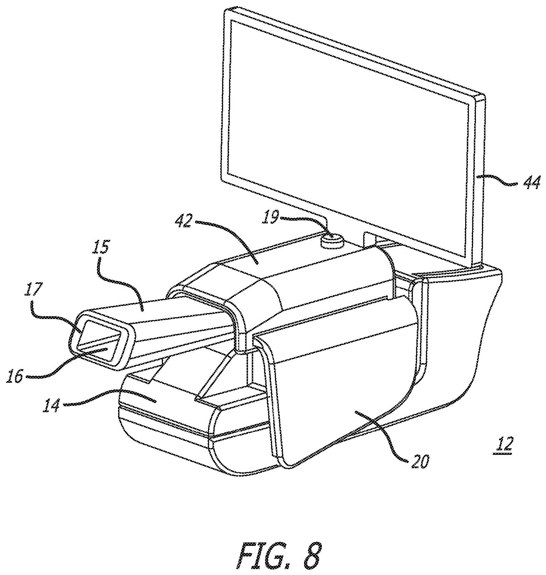

FIG. 8 depicts an isometric view of an alternate embodiment of a wireless dry powder detecting and sensing inhalation system **12** wherein the inhaler accessory apparatus **42** is shown mounted on an inhaler **14** and configured with a display screen **44** integrally configured on the body of the accessory apparatus **42** so that the patient can visualize the inhalation maneuver concurrently with his/her inhalation effort. In this embodiment, the inhaler accessory apparatus **42** comprises an electronic board **30** as shown in FIGs. 4 and 5 wherein the signal information relating to the inhaler is processed in the microprocessor **32** and the resultant processed information is communicated to the display screen **44** and presented preferably as a graphical display compared to one or more predetermined criteria for the inhaler used. This graph and associated data points are preferably stored locally on electronic board **30** but can also be stored remotely. In this and other embodiments, the predetermined criteria for an inhaler depends on the inhaler and medicament being used. In some embodiments and shown in the figures herewith, the criteria used is as indicated above as peak inspiratory pressure, emitted dose and the like.

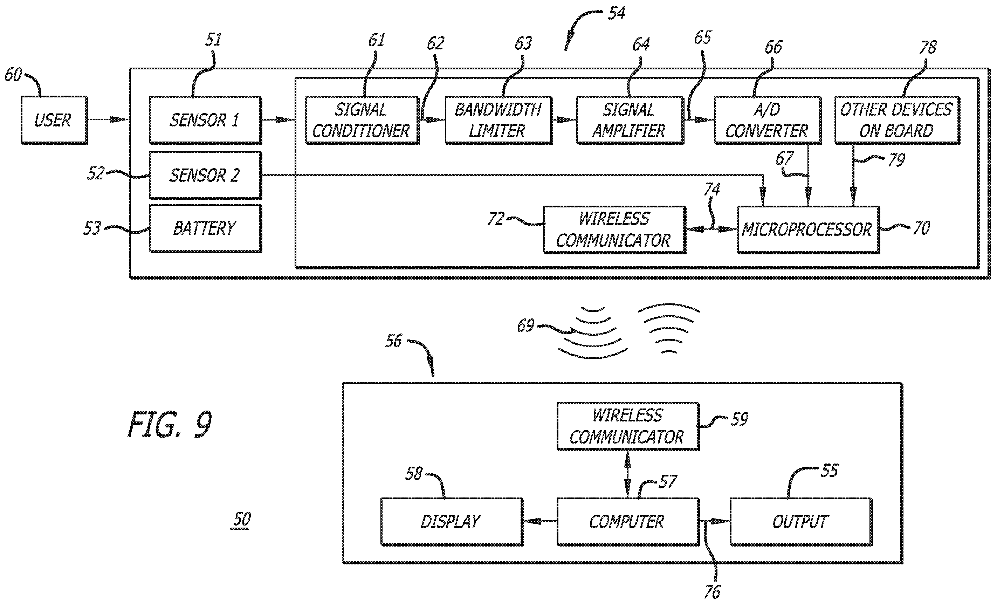

FIGs. 9, 10 and 11 illustrate various operational embodiments of the inhalation detection and monitoring system shown in FIGs. 1-8. FIG. 9 illustrates a block diagram of an overall embodiment of a wireless detection and monitoring system **50** disclosed herein. In FIG. 9, system **50** comprises two components, accessory apparatus **54** and processing system **56.** In this embodiment, the inhaler accessory apparatus **54** comprises an electronic board having two sensors **51, 52,** battery **53** a microprocessor **70** and a wireless communicator or transceiver **72.** Analog sensor **51** and digital sensor **52,** are placed so that they are in close proximity to the inhaler airflow conduits so as to be able to detect a sound signal or a pressure differential in the inhaler **14** when inhalation detection and monitoring systems **10, 12** are actuated or turned on. The system is powered on by depressing actuator button **19, 26** which is connected to a power source, such as battery **53** that also provides power to the system. Alternatively, the system can be powered by a wire such as a USB port. Sensors **51, 52** are preferably placed at any point within or proximate to the air conduit of inhaler accessory device **18, 24.** In some example embodiments, sensor **18, 24** can be placed in the air conduit within body **20** of the accessory device or near the mouthpiece **15** of the inhaler being used.

Processing system **56** can include a PDA, tablet, mobile telephone, or computer **57,** display **58,** wireless communicator **59** and output **55** which can be in the form of digital storage, a web interface, a print out or an email or the like. It should be appreciated by one skilled in the art that the display **58,** wireless communicator **59** and output **55** could simply reside within the PDA/tablet/mobile phone/computer **57** rather than being separate elements. In this example embodiment, a user can activate inhaler accessory apparatus **54** by depressing a power button, for example button **19** on apparatus **10,** with processing system **56** also activated. Computer **57** preferably includes an algorithm in the form of a software application or program designed to collect and display inhalation effort. When the software program integrated with computer **57** is initiated, a start signal appears on display **58.** With the system activated, a user's inhalation **60** generates a pressure drop in inhaler training device **50,** which is transduced to an electrical signal by one or more of sensors **51, 52.** In this embodiment, the sensors **51, 52** can be a pressure, flow, sound, optical, gas, humidity, or temperature transducer that is either analog or digital. Electrical signal generated from sensor **51** is then transmitted to signal conditioner **61** to remove unwanted portions of signals, such as signal noise. Conditioned electrical signal **62** is then transmitted to bandwidth limiter **63** to reduce the frequency of the signal to a desired range to reduce and select the data needing to be analyzed and the signal is then transmitted to a signal amplifier **64** and in signal amplifier **64,** the selected signal can be amplified to a predetermined voltage range, and transmitted as amplified signal **65.** Amplified signal **65** is then converted to digital signal **67** through analog to digital converter **66.** It should be appreciated by one skilled in the art that certain "smart" sensors can be used which integrate certain of the conditioning, filtering, amplifying and converting functionality into the sensor itself. Therefore, any reference to these subsequent elements in this specification can be replaced by use of such integrated sensors. Digital signal **67** is then received by microprocessor **70** and is transmitted into wireless communicator or transceiver **72** designed for transmission using a wireless technology standard such as Bluetooth through connection **74** for transmission to computer **57,** having wireless communicator **59** for receiving wireless (e.g., Bluetooth) signal **69.** A software program built/programmed into microprocessor **70** or computer **57** facilitates basic functionality in the inhaler accessory apparatus including advertising wireless presence, linking to wireless communicator or transceiver **59** and passing data from element to element and over wireless signal **69.** The program also converts electrical signals from sensor **1, 2** to a pressure value which can be displayed graphically in display **58.** Display **58** can be a screen comprising LED, OLED, LCD, touch screen, or other interactive display. In certain embodiments, a baseline curve for the user is stored in the system **50** and provided on the display **58** along with the inhalation signal information. The baseline curve is indicative of the level of performance for an inhaler type to deliver a substantially accurate dose to a patient as measured using an inhaler training device **10** as a reference standard to guide the user's inhalation maneuver. Therefore, during an inhalation, a user can visually compare his/her inhalation maneuver to the baseline standard. It is possible to omit the medicament from the inhaler during training of the user so that the medicament is not wasted on failed inhalation maneuvers. In this manner, the user can alter his/her inhalation effort to conform to the requirements of the standard when the drug is actually inhaled. The displayed data for each inhalation performed by a subject can be saved via second connection **76** to output **55,** wherein the data can be stored or transferred accordingly. For example, output **55** can be in the form of a disc drive or flash drive or printer, or transmitted via email or text to a physician for review or further training as needed. In some embodiments, signals from an inhalation training device can be transmitted to the computer/PDA/mobile/tablet and signals from the computer/PDA/mobile/tablet can be received by the inhalation training device, thereby establishing a two way communication between the two components. For instance, a user can input into computer **57** certain information such as patient number, dose strength, comments on condition, etc. In this and other embodiments, sensor **52** is a digital sensor or a sensor that can produce a digital output. It can be an accelerometer, a Doppler sensor, a luxometer or a laser and signal detected can be transmitted directly to the on-board microprocessor and analyzed, processed and transmitted thereafter. Signal information in the microprocessor can be analyzed and processed using algorithms, which converts the data, for example, into a pressure versus time curve using a graphical interface that can be displayed. Signals from sensor **52** can carry information relating to flow, pressure differential, and the like that is different from the signal in sensor **51** if both are employed.

Further, other on-board devices **78** can send data to and receive data from microprocessor **70** through one or more cable **79.** For example, other on-board devices can include digital output sensors, temperature sensors, light emitting diodes (LEDs), sound warning devices, and other on-board sensors. These on-board devices can be used to output pass/fail criteria of an inhalation maneuver with an LED light or audible indicator of such pass/fail. Temperature, humidity or other environmental data can be used to determine the environment in which the inhaler was used.

For sensor **51** output, following the signal amplification, amplified signal **65** alternatively can be directly sent to computer **57** via wireless communicator **72** and the computer can implement the analog to digital conversion and other required analysis steps.

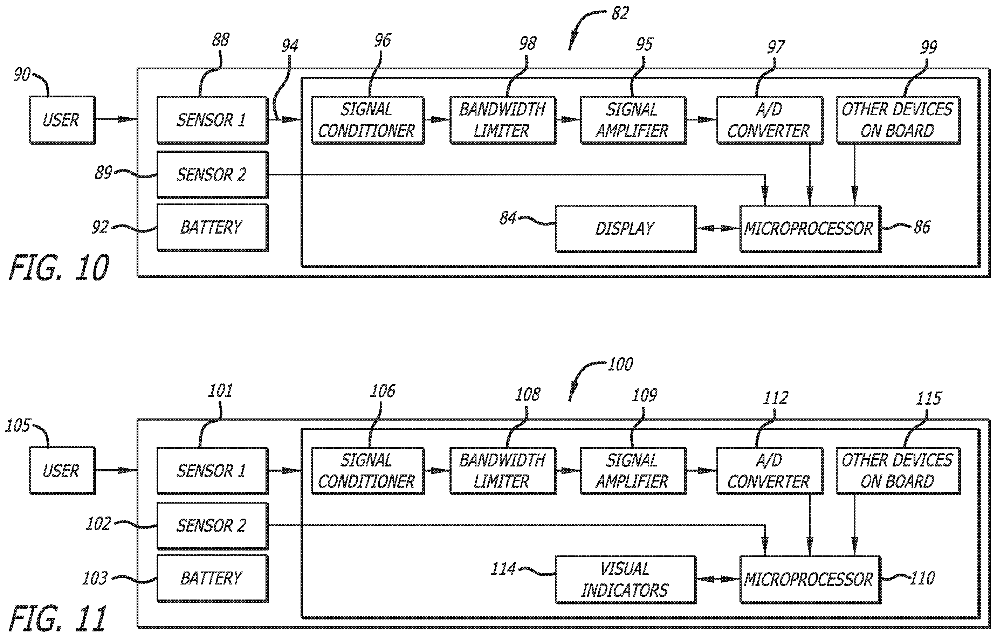

FIG. 10 illustrates a block diagram of an embodiment of a detection and monitoring system disclosed in FIG. 8 with integrated display. Inhalation detecting and sensing apparatus **82** comprises an inhaler accessory apparatus comprising an on-board electronic system with a built-in display **84,** a microprocessor **86;** an analog sensor **88** and a digital sensor **89.** The system in use is actuated by the user **90** depressing actuator button **19** with power supplied by battery **92.** When the user inhales through an inhaler adapted with inhaler accessory apparatus comprising the on-board electronic system **82,** one or more of the sensors **88, 89** generates a signal which is transmitted to the microprocessor **86.** For example, an acoustic sensor or a microphone **88** can be used to generate an electrical signal **94** which is transmitted to a signal conditioner **96** to remove excess noise and thereafter, the electrical signals are sent to a bandwidth limiter **98** to reduce the frequency of the signal to a desired range to reduce the data needing to be analyzed and the signal then is transmitted to a signal amplifier **95,** wherein in the signal is amplified and transmitted to an analog to digital converter **97** and the digital signal is communicated to an on-board microprocessor **86** for analysis and converting the information to a graph and sent to display **84** for visualizing. Sensor **89** which is a digital sensor can be used alternatively or in conjunction with sensor **88** to detect signals and generate a set of signals for transmission to microprocessor **86,** wherein the signal is analyzed, stored and sent to display **82** as well. Other devices, including other sensors **99** can also be included to detect other parameters of the inhaler or the system.

FIG. 11 illustrates a block diagram of an embodiment of a detection and monitoring system **100** disclosed herein with visual indicators of performance rather than an integrated display showing the operational parts of the system. In this embodiment, two sensors are provided, analog sensor **101** and digital sensor 102. Upon activation of the system **100** powered by battery **103,** a patient/user **105** inhales generating signals such as sound from airflow traveling through the inhaler conduits. Sensors **101, 102** are activated and establish signals from the inhaler and relay the signals downstream; sensor **102** can be a Doppler which can receive, for example, output signals from airflow detection can be either analog or digital. If output signals from sensor **102** are digital, they are transmitted directly to microprocessor **110** for analysis and processing of the incoming information. Concurrently, sensor **101** generates electrical signals, which are sensed in the inhaler, through to signal conditioner **106** to remove excess noise, then the conditioned signals are transmitted to bandwidth limiter **108** for selecting the data to be analyzed. The limited signals are then transmitted to a signal amplifier **109** wherein the signal is amplified and relayed to analog to digital converter **112.** The signal received is then converted to a digital signal and transmitted to the on-board microprocessor for analysis and processing with an algorithm which converts the data into, for example, a visual or light signal and can be displayed as a visual indicator, such as a green light or red light to indicate if the patient's inhalation effort "passed," meaning an inhalation with appropriate effort to deliver the dry powder dose, or if the patient's inhalation effort was insufficient to deliver a dry powder dose from the inhaler tested. In this embodiment, other on-board devices **115** can be integrated in the circuit, such as other sensors, or signal conditioners, amplifiers and A/D converters depending in the types of sensors use. For example, an inhaler accessory apparatus can have two or more analog sensors and therefore, the electrical signals must go through an A/D converter prior to be transmitted to a microprocessor for analysis and processing of the information. In alternate embodiments, digital sensors can be used which output signals can directly communicate with the microprocessor **86, 110.**

In other embodiments, an inhaler accessory apparatus can have one or more than one sensor, including a temperature sensor, laser beam, Doppler sensor, luxometer, color sensors, text recognition, RFID, optical character recognition, optical identification, pattern recognition, which output signal can be, for example, if not a digital signal output, an analog signal output that must be converted into digital signals for further analysis and processing once they reach the microprocessor. These sensors are preferably included on the inhaler accessory apparatus to identify what medicament is loaded in the inhaler to be administered and what cartridge type or dosage of such medicament is loaded in the inhaler.

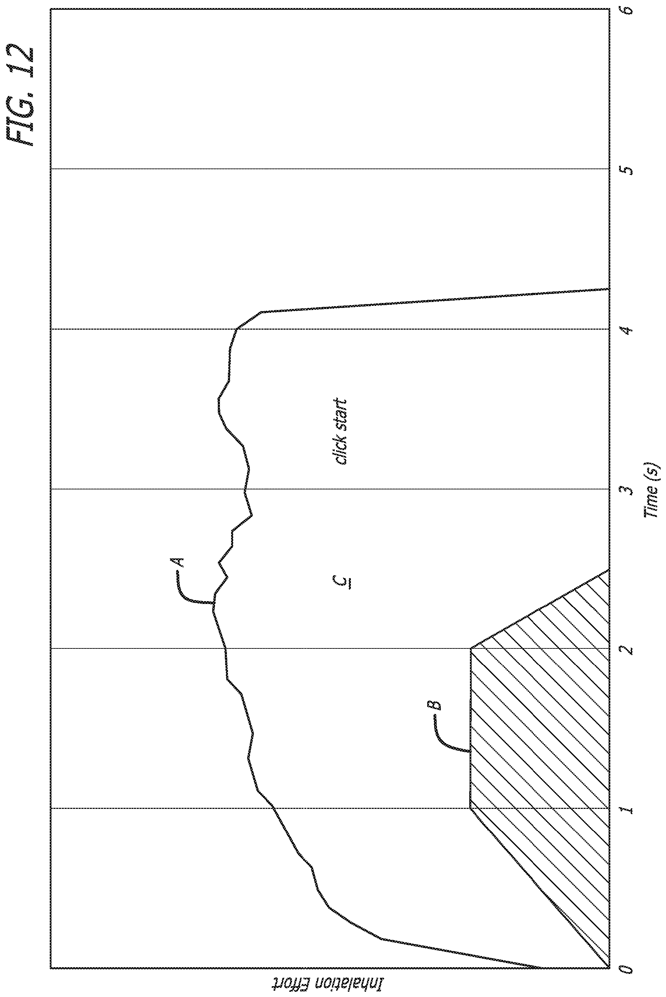

FIG. 12 shows a screen shot of a tablet/computer/PDA/phone **57** of processing system **56** in FIG. 9. The computer **57** is used to communicate remotely with the inhaler accessory apparatus **54** using Bluetooth or another remote wireless technology, wherein the inhaler accessory apparatus **54** is adapted to an inhaler and the subject is asked to inhale through the mouthpiece of the inhaler when the system is activated. The resultant graph on the screen as shown in FIG. 12 is plotted in response to the inhalation maneuver as inhalation effort on the y-axis, taking into account sensor (e.g., pressure) measurements and flow versus time in seconds on the x-axis. The subject's inhalation effort is represented by the curve A above the trapezoid B figure at the base of the graph. The trapezoid B outer limits (i.e., above the area) is interpreted as indicating the threshold or minimum inhalation effort a subject must exert to be able to effectively and consistently inhale a powder dose from the inhaler used to empty the contents of the powder in the inhaler in taking a dose. An identification sensor, such as those described herein located in the inhaler accessory apparatus, detects and transmits data associated with the inhaler, medicament type, dosage, lot, expiration, etc. Such data is processed to identify the corresponding threshold data for user indication. The trapezoid also indicates the minimal characteristic criteria the inhaler exhibits or effort the inhaler requires to deliver a dry powder dose consistently, which delivery is greater than 90% to the patient. FIG. 12 graphically illustrates the example display of an inhalation maneuver performed by a subject who was asked to inhale deeply and is allowed to see the display screen on a tablet while performing the inhalation. As can be seen by such curve, the subject performed entirely within acceptable values in region **A.**

Additionally, FIG. 12 depicts a baseline inhalation performance standard for inhaler accessory apparatus **10** and the medicament identification. The user detected curve **A** can be bordered by a warning region just above region **B** and an acceptable or preferred region **C** above the warning region. Regions **B** and **C** and the warning region can be provided in different colors to facilitate discernment of regions in monitoring an individual's performance during an inhalation. Region **B** can be, for example, depicted in red, indicating that the inhalation maneuver did not meet the baseline requirement; therefore, the delivery system would not be optimal to deliver a medicament effectively. The warning region can be depicted in yellow indicating a warning that the inhalation maneuver is nearing the unacceptable performance effort. Preferred region **C** can be depicted in green indicating that the inhalation performance is in the acceptable efforts to effectively deliver a medicament. This displayed information detected from one or more sensors in the apparatus can be used by a clinician, physician or user to determine whether proper dosing occurred or it can be used to train the user on how much effort is needed to ensure proper dosing of the medicament.

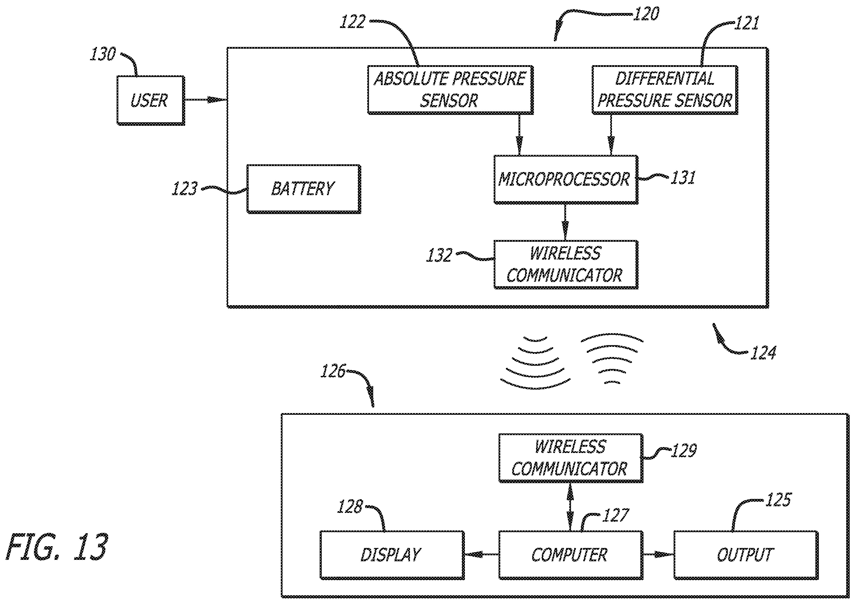

FIGs. 13, 14 and 15 illustrate various operational embodiments of the inhalation detection and monitoring system shown in FIGs. 1-8. FIG. 13 illustrates a block diagram of an overall embodiment of a wireless detection and monitoring system as disclosed herein. In FIG. 13, system **120** comprises two components, inhaler training device or accessory apparatus **124** and processing system **126.** Processing system **126** can include a PDA, mobile telephone, or computer **127,** display **128,** wireless communicator **129** and output **125** which can be in the form of digital storage, a web interface, a print out or the like. In this example embodiment, a user can activate inhaler training device or apparatus **120** by depressing a power button, for example, button **19** on training device **10,** with processing system **126** also activated. When the software program integrated with computer **127** is initiated, a start signal appears on display **128.** In this embodiment, the accessory apparatus **120** comprises an electronic board having preferably two pressure sensors **121** and **122** placed so that they are in close proximity to the inhaler airflow conduits so as to be able to detect the differential pressure from the inhaler and the absolute pressure of the environment from the inhaler **14** when apparatus **10, 12** is actuated or turned on by depressing actuator button **19, 26,** which is connected to a power source, such as battery **123** that also provides power to the system. With the system activated, a user's inhalation **130** generates a pressure drop in inhaler training device **120,** which is measured by sensor **121.** Absolute pressure sensor **122** provides a data or signal used to correct the differential pressure reading for atmospheric conditions.

In this embodiment, sensors **121** and **122** are pressure sensors that are digital. Signals generated by sensors **121** and **122** are then transmitted to microprocessor **131** and into wireless communicator **132.** A software program built into/programmed into microprocessor **131** or computer **127** converts signals generated by sensors **121** and **122** to a (corrected) pressure value which can be displayed graphically in display **58,** which can be a screen comprising LED, OLED, LCD, touch screen, or other interactive display.

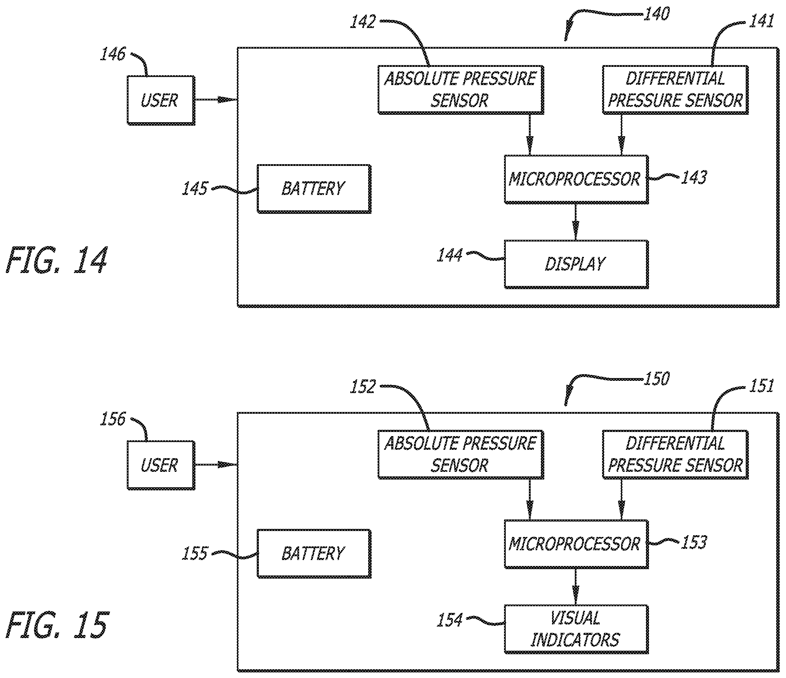

FIG. 14 illustrates a block diagram of an embodiment of a detection and monitoring system disclosed herein. The inhalation detecting and sensing apparatus comprises an inhaler accessory apparatus comprising an on-board electronic system **140** with a built-in or integrated display **144,** a microprocessor **143,** and pressure sensors **141** and **142.** The system in use is actuated by user **146** with power supplied by battery **145.** With the system activated, a user's inhalation **146** generates a pressure drop in inhaler training device **140,** which is measured by sensor **141.** Absolute pressure sensor **142** provides a data or signal used to correct the differential pressure reading for atmospheric conditions. In this embodiment, sensors **141** and **142** are pressure sensors that are digital. If analog sensors are implemented, additional circuit elements would be necessary for conditioning, filtering, amplifying and/or converting the signals as discussed hereinabove. Signals generated by pressure sensors **141** and **142** are then transmitted to microprocessor **143.** A software program built into/programmed into microprocessor **143** converts signals generated by sensors **141** and **142** to a (corrected) pressure value which can be displayed graphically in display **144,** which can be a screen comprising LED, OLED, LCD, touch screen, or other interactive display.

FIG. 15 illustrates a block diagram of an embodiment of a detection and monitoring system disclosed herein. Inhalation detecting and sensing apparatus comprises an inhaler accessory apparatus comprising an on-board electronic system **150** with integrated visual indicators **154,** a microprocessor **153;** and pressure sensors **151** and **152.** The system in use is actuated by user **156** with power supplied by battery **155.** With the system activated, a user's inhalation **156** generates a pressure drop in inhaler training device **150,** which is measured by sensor **151.** Absolute pressure sensor **152** provides a data or signal used to correct the differential pressure reading for atmospheric conditions. In this embodiment, sensors **151** and **152** are pressure sensors that are digital. If analog sensors are implemented, additional circuit elements would be necessary for conditioning, filtering, amplifying and/or converting the signals as discussed hereinabove. Signals generated by pressure sensors **151** and **152** are then transmitted to microprocessor **153.** A software program built into/programmed into microprocessor **153** converts signals generated by sensors **151** and **152** to a (corrected) pressure value which can be used to activate visual indicators **154** which can be used to indicate a correct inhalation or other information.

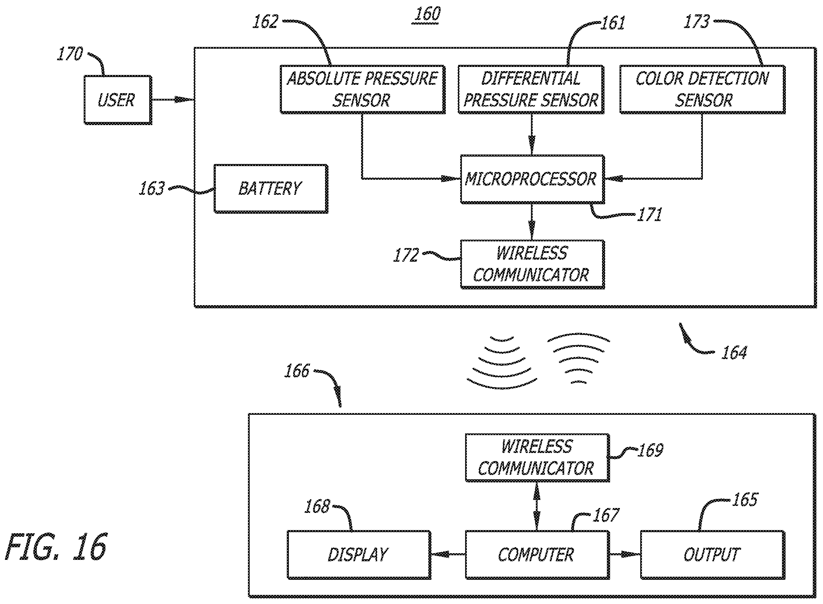

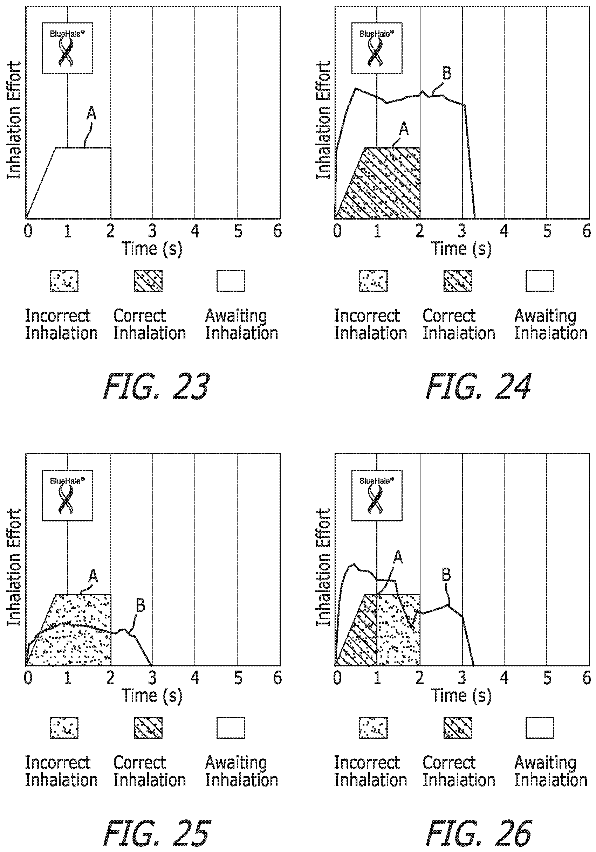

FIG. 16 further illustrates a block diagram for, for example, an inhaler training device, such as apparatus **10,** showing further various operational component parts. In FIG. 16, system **160** comprises two components, inhaler training device or accessory apparatus **164** and processing system **166.** Processing system **166** includes a tablet, PDA, mobile/smart telephone, smart watch, smart glasses, or computer **167,** display **168,** wireless communicator **169** and output **165** which can be in the form of digital storage, a web interface, a print out or the like. In this example embodiment, a user can activate inhaler training device **160** by depressing a power button, for example, button **19** on training device **10,** with processing system **160** also activated. When the software program integrated with computer **167** is initiated, a start signal appears on display **168.** With the system activated, a user's inhalation **170** generates a pressure drop in inhaler training device **160,** which is measured by sensor **161.** In this embodiment, sensors **161** and **162** are pressure sensors that are digital. Signals generated by color detection sensor **173** and pressure sensors **161** and **162** are then transmitted to microprocessor **171** and into wireless communicator **172.** A software program built into/programmed into microprocessor **171** or computer **167** converts signals generated by color detection sensor **173** and sensors **161** and **162** to a cartridge information value and a pressure value, respectively, which can be displayed graphically in display **168,** which can be a screen comprising LED, OLED, LCD, touch screen, or other interactive display. The cartridge information value can be used to provide the limits to powder dose efficacy and plot as the trapezoid B, or other threshold indication on a graphic display (for example, FIGs. 23, 24, 25 and 26). As referenced in earlier embodiments, in addition to color detection devices, other devices on the apparatus board can include laser, RFID, pattern or text/character readers or sensors with connections to the microprocessor to otherwise identify the inhaler, drug or cartridge/packaging of the substance/drug. These sensors/readers function to provide data to the system and microprocessor relating to medicament, substance, packaging, dosing, inhaler, etc. so that the corresponding data can be retrieved from storage and used as data points on any visual, audible or other indicator including a graph presented to the user. As an example, certain cartridges or other packaging can be color coded or include encrypted or encoded text, RFID indicating specific information about them including lot, expiration date, dosages, etc. A reader or sensor that can detect the code and send the corresponding data to the microprocessor for use in calculations, identified actions, and data presentation. Perhaps a certain color package indicates the use of a drug dose that requires greater effort to inhale properly. In this case, the accessory device or system will identify the proper color through the sensor/reader and use the proper data for instruction to the user.

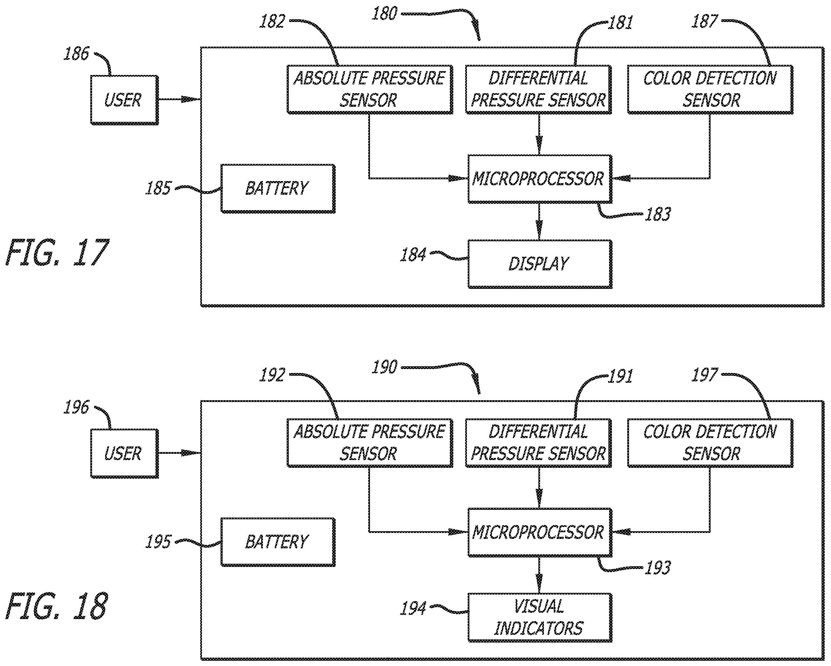

FIG. 17 illustrates a block diagram of an embodiment of a detection and monitoring system disclosed herein. Inhalation detecting and sensing apparatus **180** comprises an inhaler accessory apparatus comprising an on-board electronic system with a built-in or integrated display **184,** a microprocessor **183,** color detection sensor **187** and pressure sensors **181** (differential) and **182** (absolute). The system in use is actuated by user **186** with power supplied by battery **185.** With the system activated, a user's inhalation **186** generates a pressure drop in inhaler accessory apparatus, which is measured by sensor **181.** Absolute pressure sensor **182** provides a data or signal used to correct the differential pressure reading for atmospheric conditions. In this embodiment, sensors **181** and **182** are pressure sensors that are digital. Signals generated by color detection sensor **187** and pressure sensors **181** and **182** are then transmitted to microprocessor **183.** A software program built into/programmed into microprocessor **183** converts signals generated color detection sensor **187** and sensors **181** and **182** to a cartridge information value and a (corrected) pressure value which can be displayed graphically in display **184,** which can be a screen comprising LED, OLED, LCD, touch screen, or other interactive display.

FIG. 18 illustrates a block diagram of an embodiment of a detection and monitoring system disclosed herein. Inhalation detecting and sensing apparatus **190** comprises an inhaler accessory apparatus comprising an on-board electronic system with integrated visual indicators **194,** a microprocessor **193,** color detection sensor **197** and pressure sensors **191** (differential) and **192** (absolute). The system in use is actuated by user **196** with power supplied by battery **195.** With the system activated, a user's inhalation **196** generates a pressure drop in inhaler accessory apparatus, which is measured by sensor **191.** Absolute pressure sensor **192** provides a data or signal used to correct the differential pressure reading for atmospheric conditions. In this embodiment, sensors **191** and **192** are pressure sensors that are digital. Signals generated by color detection sensor **197** and pressure sensors **191** and **192** are then transmitted to microprocessor **193.** A software program built into/programmed into microprocessor **193** converts signals generated from color detection sensor **197** and sensors **191** and **192** to a cartridge information value and a (corrected) pressure value, respectively, which can be used to activate visual indicators **194** which can be used to indicate a fail or correct/acceptable inhalation or other information.

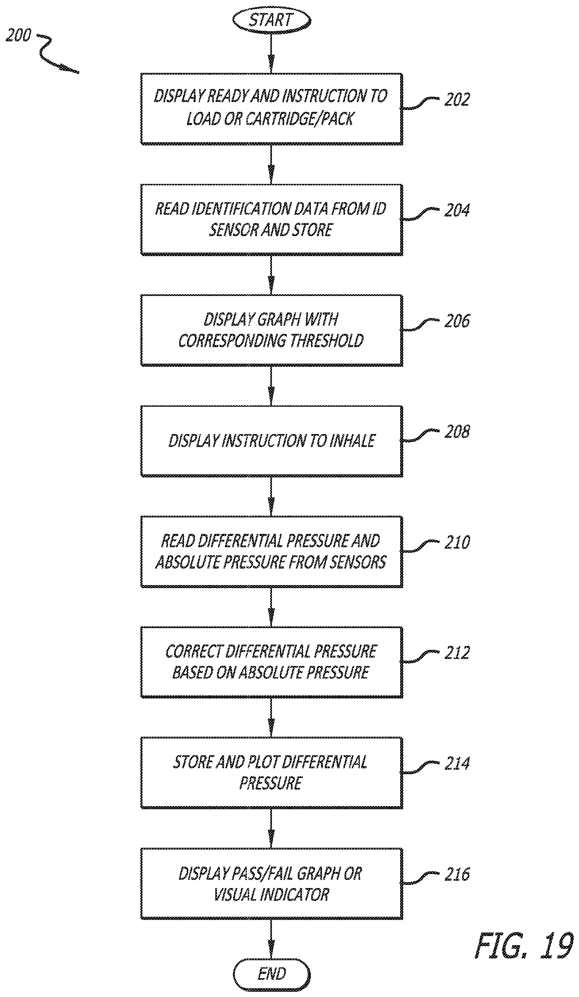

FIG. 19 illustrates a flowchart diagram of an embodiment of a method **200** of detecting, monitoring and training an inhalation subject according to the system disclosed in FIG. 16. When a user is to use the apparatus and system, he or she actuates the system by depressing the actuator on the inhaler accessory apparatus to start. Next in step **202,** the wireless communicator of the inhaler accessory apparatus links using, for example, standard Bluetooth technology, to the user's smart phone and an application on the phone displays a ready message and instructs the user to load the related inhaler with which the apparatus is engaged or provide a cartridge or other disposable package of the substance to be inhaled into the inhaler. In step **204,** after such loading occurs, the identification sensor, in this case a color detection sensor **173** determines the color of the cartridge and stores it in data storage on the accessory apparatus board or transmits it via Bluetooth to the processing system **166** (phone) for storage. In step **206,** the application displays a graph with the corresponding threshold data points provided based on the color detection. The user is next instructed to inhale in step **208** through use of some visual, audible or screen based message. In step **210,** during an inhalation maneuver, the sensors **161, 162** read the pressure drop. Next, step **212** shows a correction step taken by the system based on the atmospheric conditions which occurs substantially simultaneously with step **210** or just thereafter. In either case, the pressure data is stored as above and preferably plotted on a graph in step 214. In step **216,** the inhalation maneuver graph is displayed to the user along with the threshold graph showing either a pass (successful inhalation) or a fail (unsuccessful inhalation). At that time, the user can depress the actuator to end the program and the data remains stored in the output **165** for future use.

In some example embodiments disclosed herein, one or more key parameters can define an acceptable inhalation maneuver, including, total inhalation time, peak inspiratory pressure, time to peak inspiratory pressure and average pressure from peak to about 75% of the total inhalation time. In certain embodiments, the total inhalation time is 0.1 up to 5 seconds, or between 0.1 and 3 seconds. In some embodiments, the inhalation time can be greater than 5 seconds, the peak differential inspiratory pressure can be greater than about 2 kPa, or from between 2 and 6 kPa. In some embodiments the peak inspiratory pressure can be greater than about 6 kPa; time to peak inspiratory pressure can be less than about 1.1 seconds and the average pressure from peak differential inhalation to 75% of total inhalation time is about 4 kPa. These values are representative of values for an inhalation monitoring system **10, 12** and apparatus **18** and related algorithms/programs used for training and monitoring inhalations with a high resistance dry powder inhaler. They can be modified for alternate inhaler training devices, depending on the performance parameters required for optimal delivery of the medicament of the inhaler, including resistance.

In another example embodiment, a dry powder inhaler can be provided with a sensing and/or monitoring device which can monitor and/or sense signals generated by or within a dry powder inhaler during an inhalation maneuver by a patient. Dry powder inhalers can be provided with a sensor device either integrated into the device or attached thereto. Alternatively, in an example embodiment, accessory apparatus **18, 24** can be provided as an integral part of dry powder inhaler on mouthpiece or housing as desired.

In alternate embodiments, the inhaler accessory device **18, 24** is a mountable/detachable sensing and monitoring device that can disengage from the inhaler and is provided in the form of a jacket or cap, wherein detachable sensing and monitoring device can be provided as a detachable part that can adapt to a dry powder inhaler, in particular, for wireless communication so that the subject using the device has easier access and movement. In this embodiment, the jacket is manufactured as a separate, detachable device comprising on-board electronics including one, or more microprocessors, wireless transceivers, A/D converters, sensors (such as a pressure sensor or a microphone) which can detect signals and being capable of storing, transmitting or displaying the signals.

When using acoustic sensors, sound waves emanating from the inhaler in use with or without a dry powder are detected by the microphone and the signals can be analyzed and correlated to time of powder discharge in the presence of a dry powder, airflow rate, end of powder discharge during an inhalation maneuver, temperature within the inhaler pathway, and the like, depending on the type of sensor used. For example, an increase in sound can be correlated to an increase in flow rate through the device, and/or powder particles collisions in the air stream during delivery.

A sensor such as a microphone, as a result of its small size, can be placed anywhere in the inhaler. In embodiments wherein the sensor is a pressure transducer, the sensor can be placed within an air conduit passing through one of the inhaler compartments. The sensors can be provided, for example, in an air conduit on or within the inhaler or provided as a separate, detachable part as an accessory to the inhaler with a shape or configuration that can be adapted to the inhaler to which is to be adapted, and can include a cap, a jacket, sleeve or a saddle-like configuration that can be adapted or mounted to the inhaler.

For the detachable embodiments, the sensing and monitoring accessory apparatus is easy and inexpensive to manufacture and can be made from plastics, and works well with high resistance dry powder inhalers. In some embodiments, the sensor can be any sensor, for example, a thermocouple wire, a pressure transducer, an analog sensor, a microphone, an optical sensor, color sensor, including electromagnetic radiation sensors including spectral sensor, infrared sensors and visible spectral sensors, a gas sensor, or any sensor that can detect signals generated within an inhaler. The sensors described herein can be adapted to communicate or transmit signals with a transceiver device or the signals can be transmitted or stored using wire connection to an analog to digital converter prior to transmitting this signals to a microprocessor.

Alternatively, an analog to digital converter is provided within the inhaler device and resulting digital data is transferred out of the device directly. The signals provided by the sensors described herein can be in several forms including sound generated in an inhaler by airflow passing through the air conduits and/or powder particles collisions entrained in the air flow pathway and pressure drops detected proximate to the airflow pathway due to the inhalation maneuver. Signals generated from the inhaler can be detected by the sensors and stored, transmitted or displayed. Other types of signals that can be detected by the system are text, color, encryptions or codes, which can be detected by light beams, laser beams, and Doppler sensors which are, preferably, integrated into the electronic board. Data can be generated from the signals and qualitatively and/or quantitatively analyzed. In this manner, measurements can be made including time of dose release, amount of dose, type of dose, time of dose, and the like. Further, these signals, for example, can be associated with identification of the patient, the medicament type and dosage, the inhaler or otherwise and can be used to model the data requirements for proper inhalation and facilitate training of the inhaler user.

In one example embodiment, a sensing and monitoring system for an inhaler includes an accessory apparatus structurally configured to be adapted to an inhaler; at least one sensor, a microprocessor, an optional analog to digital converter; and a data storage medium. The data storage medium includes a disc drive, a DVD, CD-ROM, a server, a flash card or drive, memory card, and the like and includes a set of machine-readable instructions that are executable by a microprocessor or other processing device to implement an algorithm. The algorithm, when run, initiates the steps of generating a logical sub-system generation number derived from detected signals; saving the logical sub-system generation number to a data track within a logical sub-system, wherein the logical sub-system generation number and a cluster generation number in the processing device are compared; and storing and/or displaying information from the algorithm as the results from an inhalation maneuver.

In an alternate embodiment, a dry powder inhaler can be provided with a sensing and/or monitoring device which can monitor and/or sense signals generated by or within a dry powder inhaler during an inhalation maneuver by a patient. Dry powder inhalers can be provided with a sensor device either integrated into the device or attached thereto. Alternatively, the accessory apparatus can be provided as an integral part of dry powder inhaler on mouthpiece or housing as desired.

In the alternate embodiment, the inhaler accessory device is a mountable/detachable sensing and monitoring device that can disengage from the inhaler and is in the form of a jacket or cap, wherein detachable sensing and monitoring device can be provided as a detachable part that can adapt to a dry powder inhaler, in particular, for wireless communication so that the subject using the device has easier access and movement. In this embodiment, the jacket/inhaler accessory device is manufactured as a separate, detachable device comprising on board electronics for processing information, including one or more microprocessors, wireless transceivers, A/D converters, sensors which can detect signals, such as color signals and being capable of storing, transmitting or displaying the signals.

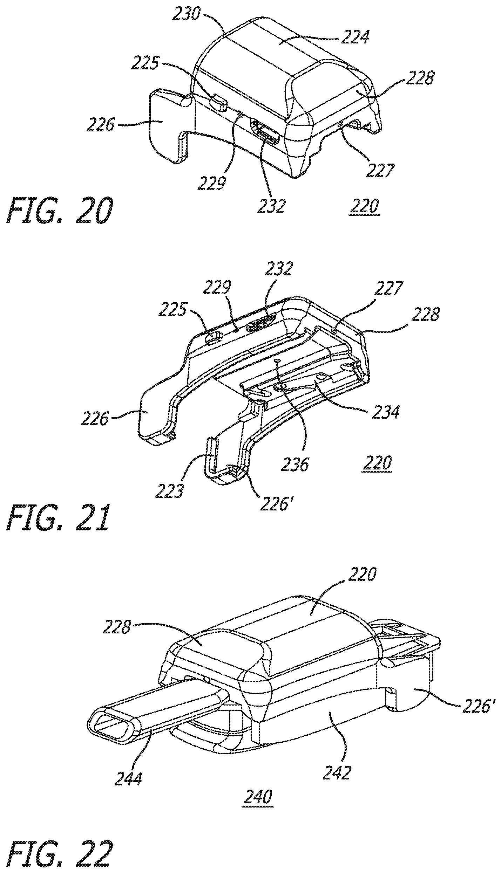

An example embodiment is illustrated in FIGs. 20-22. Inhalation detecting and monitoring apparatus comprises a detachable inhaler accessory apparatus **220** for adapting to an inhaler and having a proximal end **228,** distal end **230** and comprising, an on-board electronic system, the system comprising a body **224** comprising two members, a top member and a bottom member configured for adapting to one another and a circuit board comprising the electronic system placed in between the top and the bottom members and held together by securing mechanism such as screws; the top member comprises arm extensions **226, 226'** which project downwardly for securely adapting to an inhaler distal end away from a mouthpiece and comprising securing mechanisms **223.** The circuit board comprising a microprocessor comprising a transceiver; a differential pressure gauge; an absolute pressure gauge; and an accelerometer for determining spatial orientation of the accessory assembly in use. FIG 21 shows the undersurface of body **224,** bottom member, which comprises pressure equalization channel **234,** which is configured between the absolute pressure sensor and the differential pressure sensor, which equalizes pressure within the absolute pressure sensor and the differential pressure sensor in use, and thus facilitating pneumatic communication between the absolute pressure sensor and the differential pressure sensor. In one embodiment, the differential pressure sensor/gauge is configured to be place in close proximity to an inhaler air flow pathway so that it communicates with an air pathway of the inhaler, and absolute pressure sensor/gauge is configured anywhere on the device. In a prefer embodiment, the absolute pressure sensor is configured within the circuit board and communicates with the differential pressure sensor through the pressure equalization channel **234.** A critical finding of the instant system was that when communication between the absolute and differential sensors was impeded, the inhalation detecting and monitoring apparatus did not function. Body **224** is also configured having opening **236** on its undersurface to allow access to a reset button for repowering the system should actuating button **225** fail.

Body **224** also comprises actuating (on/off) button **225,** which is connected to a power source such as a lithium battery for powering or turning off system **220;** UBS port **232** for recharging system **220** or downloading stored information; quality signal indicator **227** comprising LED lights for showing a light signal such as red (fail) or green (pass) signal; system status indicator **229** configured in proximity to actuating button **225** for indicating power/charge connector status. In specific embodiments, a sensor with at least two emitters and at least two receivers can be used depending on the complexity of the system.

In this and other embodiments, inhaler accessory device **220** comprising an accelerometer in use can detect the spatial orientation of an inhaler when accessory device **220** is mounted onto an inhaler. For example, FIG. 22 illustrates inhalation detecting and monitoring system **240** comprising inhaler **242** adapted with wireless inhaler accessory device **220.** In this embodiment, when the system is actuated, movement of the inhaler can be displayed on a screen of a mobile phone, a tablet, and the like, using an application which can tell a user the correct positioning of the inhaler. If the inhaler is used in the wrong position prior to an inhalation maneuver to deliver a dose of medication, device **220** will be depicted in the display with a bad orientation/fail (red color) or correct/proper orientation (green/white). Alternatively, haptic feedback signals, or visual signals can be generated from device **220** to signal if the proper or improper orientation of the device in use, if orientation is good or no good depending on the user's positioning of the device if device is not coupled to a PDA, computer, mobile phone or the like. In yet another embodiment, an inhalation detecting and monitoring jacket is manufactured as a separate, detachable device comprising on board electronics including one or more microprocessors, wireless transceivers, A/D converters, sensors which can detect signals and being capable of storing, transmitting or displaying the signals. In this embodiment, a software application (app) may be used on a wireless device, such as a smart phone and/or tablet rather than a desktop or laptop computer. This app may provide graphical interfacing showing a 3D model of the inhaler. The 3D model of the inhaler may provide feedback on the spatial orientation of the inhaler. For example, if the user is holding the inhaler during inhalation at an incorrect angle, the user may be alerted to the improper use by a change in the color of the 3D inhaler model on the screen, for example, the 3D model may appear as a red color. Improper use of the inhaler may lead to loss of powder contents during dosing. Alternative, a voice from the phone/table app indicates the improper positioning of the inhaler by alerting visually or audibly the incorrect orientation of the inhaler to the user.

In another embodiment, inhalation accessory apparatus comprises color sensors for detecting multiple and discrete wavelengths and comprising discrete wavelength measurement channels. The color sensors can identify, for example, the color of a capsule or identity of cartridge with dose of powder that is being used, the amount of powder in the cartridge and provide the quality of the discharged powder within the inhaler. The sensors can alert the user of these parameters by LED lights, audible/annunciations to the user or tactile feedback such as vibration. In one embodiment, a spectral sensor is used wherein the sensor can detect discrete or continuous wavelengths which can be algorithmically manipulated to determine a specific color of a cartridge or capsule. Using this type of spectral sensor one can determine what color cartridge is being used and is equated to the dosage strength of the contents of the cartridge. The information of the dosage of the cartridge can be signaled or transmitted to the user for confirmation of dose, or data can be stored in the device for comparative in a subsequent used. Additionally, the data can be analyzed via artificial intelligence algorithms to further improve the accuracy of color detection.

In particular embodiments, the inhaler accessory apparatus comprises a photo sensor, which can measure ambient light signals, including in the visible light, infrared transmission (IR), and/or ultraviolet (UV) energy. These signals can be used to determine information about cartridge use as well as powder flow through the inhaler. In one embodiment, the apparatus is equipped to detect ambient light being emitted from the inhaler or parts of the inhaler, or when the emissions are augmented by a secondary light source, including, a flash of light generated from the accessory device. In this embodiment, the signals emanating from the inhaler or parts of the inhaler are intensified for improved precision detection of the light signals. Signals generated from the inhaler under ambient or augmented conditions are characteristic of the inhaler of part of the inhaler, including the cartridge or capsule with a dose if color-coded to represent a specific active agent or amount of dosage. In one embodiment, the inhaler accessory apparatus comprises a mean for producing light, including, a light emitting diode (LED).

In one embodiment, the inhaler accessory apparatus comprises an automatic collection system that detects the onset of an inhalation maneuver as measured by the pressure sensors when the user begins an inhalation effort. In this embodiment, the pressure sensors are configured to detect pressure differential at predetermined values and activates the entire system to operate automatically. In certain embodiments, the automatic collection of signals can also automatically turn off after a period of time, which can be preset into the device. In embodiments herewith, data capture includes entirety of the inhalation maneuver and detection of the cartridge in use. In one embodiment, the inhalation effort provided by a subject is automatically measured when minimum threshold occurs.

In another embodiment, the inhaler accessory apparatus comprises a system for storing and transmitting data related to an inhaler and a cartridge in use, including, inhalation maneuvers and cartridge dosing or utilization parameters and powder flow characteristics, so that the information can be analyzed for optimization of a subject's use patterns and/or therapeutic compliance of dozing regimes. In this embodiment, the inhaler accessory apparatus monitors a subject's use and consistency of delivery of a therapeutic dose. In one embodiment, data acquired by the apparatus can be stored on the device and transmitted to a digital application where it can be analyzed for optimal therapeutic utility of a patient.

In yet another embodiment, a sensor such as a Doppler Ultrasonic sensor or "time of flight" sensor may be used to measure the amount of discharge of the powder. The Doppler sensors can detect ultrasonic diffraction signals from powder flow from an inhaler in use. The ultrasonic diffraction signals may provide feedback from the amount of powder discharged from the inhaler during an inhalation and/or the quality of the inhalation maneuver (amount of powder to be inhaled by user), which may be provided by illuminating LEDs, tactile feedback (example: vibrating) or audible/annunciating feedback to the user. For example, an LED in the jacket or tablet may illuminate a solid red LED alerting the user to inhale harder, or a blinking red LED alerting the user to inhale longer or a solid green LED alerting the user of a correct inhale. Another example is using tactile feedback in the jacket or tablet where there would be a vibration alerting the user to inhale harder or several vibrations alerting the user to inhale longer or no vibration indicating a correct inhalation. In another example, the device may annunciate the quality of the inhale to the user to particularly assist the visually impaired in administering the powder.

In training a subject to use an inhaler properly for delivering an effective dose of a powder medication by inhalation, FIGs. 23-24 illustrate examples of inhalation maneuvers performed by naïve subjects prior to independent use with an inhalation detecting and monitoring apparatus as shown in FIGs. 20-22. For training purposes, an empty cartridge or a placebo powder can be used so drug powder doses are not wasted. Screen shots from a mobile phone interactively linked to inhalation detecting and monitoring apparatus **220** by a Bluetooth transceiver, wherein there is displayed a standard baseline curve (Blank area) for measuring the subject's inhalation efforts (FIG. 23) as a measure of time in seconds.

FIG. 23 is a screen shot of the mobile phone display when device **220** is actuated and the mobile phone is also activated, and the screen shot of the mobile phone depicts a graphic user interface with area above curve A as pass and below curve A as fail prior to an inhalation. Area above curve A was determined as a standard baseline from data obtained from multiple measurements from an inhaler, which graphically illustrates the inhaler threshold parameters for effectively delivering its powders content during a single inhalation. Upon a full inhalation achieving threshold parameters, area below A is shown as hatched lines in FIG. 24 (colors can also be used) indicating that a user's inhalation which generated curve B above the standard curve is proper and thus the user's inhalation is acceptable to deliver effectively a dose of a powder contained in the inhaler.

If the patient's inhalation was performed improperly, FIGs. 25 and 26 depicts two different scenarios. FIG. 25 is a screen shot of a resultant user's inhalation, showing that the inhalation was poorly performed to deliver a dose of a powder since the user's effort fell within the standard curve. The displayed standard curve is shown in a visual dark/red color indicating a failed inhalation. In this scenario, the user inhaled for a prescribed time period, but the strength of the effort was weak. In training this subject, the user is instructed to inhale harder or with more effort until the user achieves the graphics similarly as depicted in FIG. 24. FIG. 26 is a screen shot of a mobile phone's illustrating a fail inhalation effort that was not sustained for the appropriate period of time by the user. In this scenario, the user's inhalation was proper initially, but the user was incapable of maintaining the inhalation effort for the time required for proper inhaler use. Thus, the user in this case would not be able to receive a full dose of medicament. Subsequently, the user is instructed to maintain an initial strong inhalation effort for a longer period of time until the user can generate a curve similarly as depicted in FIG. 24 and above the threshold parameters of the inhaler. In this manner, inhaler users can be trained properly to use their inhalers without assistance and dose themselves.

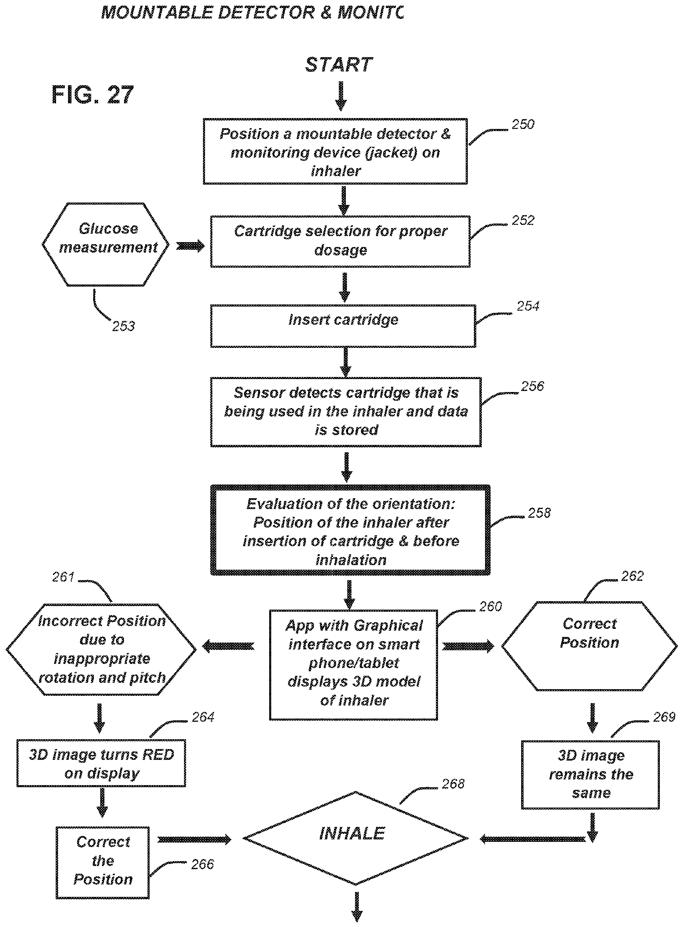

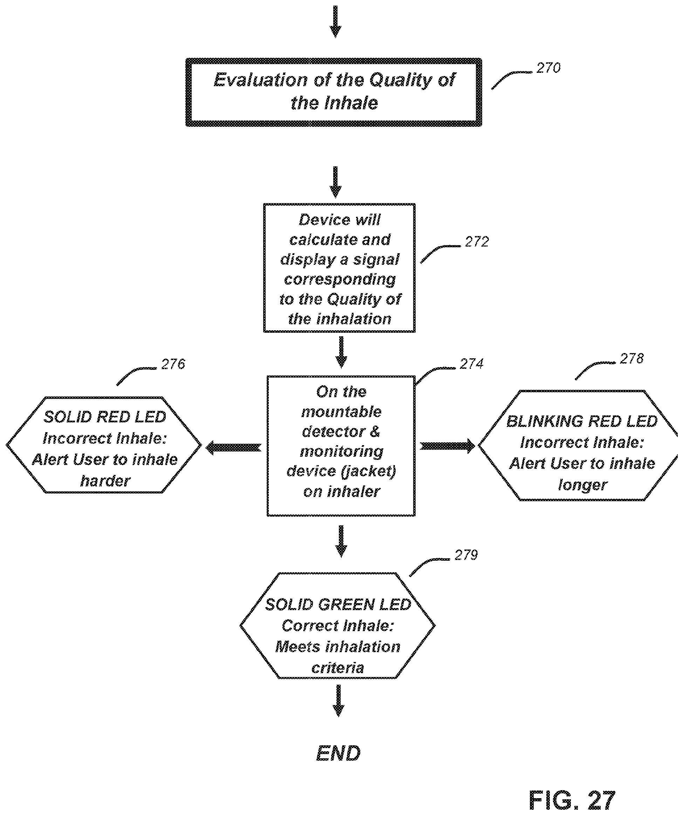

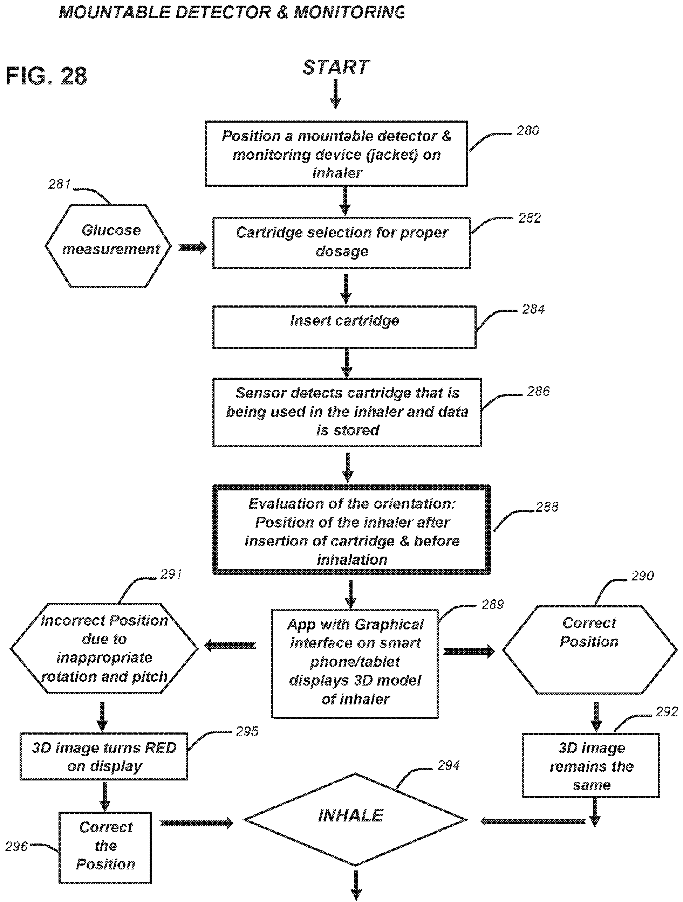

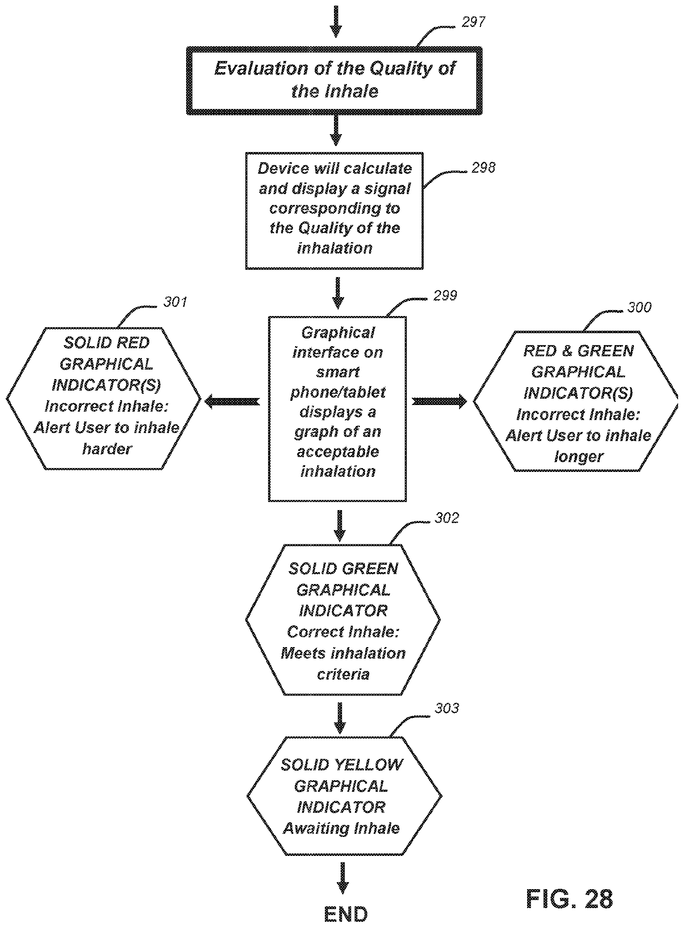

FIGs. 27 and 28 are flow charts illustrating the operational steps of the inhalation detecting and monitoring device embodiments in use. In FIG. 27, an inhaler for use with dry powder inhaler, such as the AFREZZA^{®} inhaler (MannKind Corp.), is adapted with a detachable detecting and monitoring apparatus. As depicted in FIG. 27, at the start of training, an inhalation detecting and monitoring device is assembled on an inhaler as shown in FIG.22. All devices are powered on at step **250.** Depending on the patient's food intake or the patient's blood glucose levels at step **253,** an appropriate dose of cartridge is selected from 4 units, 8 units, 12 units or 16 units at step **252** and installed into the inhaler by opening the inhaler and inserting the cartridge in the cartridge mounting area at step **254.** Once cartridge with an appropriate dose is inserted into the inhaler, the inhaler is closed in step **256.** A sensor, which may include an optical sensor or color sensor, is triggered to detect the identity of the cartridge in the cartridge mounting area of inhaler and the data is stored and transmitted to a microprocessor. Once the cartridge is detected in the inhaler, evaluation of the orientation **258** and positioning of the inhaler before inhalation is analyzed. Signals received in smart phone/tablet displays a 3D model of the inhaler shown with an acceptable color selected for proper spatial orientation is displayed in the wireless device screen, which utilizes an app displaying a graphical interface at **260.** If the inhaler is in a correct/proper special orientation for performing an inhalation at **262,** the color selected for the inhaler 3D display should remain the same **269.** If an incorrect/improper position for inhalation due to inappropriate rotation and pitch of the inhaler at **261,** the 3D image of the inhaler turns a different than the one selected for the inhaler in the correct position, such as the color red as a fail indicator **264.** If the position of the inhaler is corrected at step **266,** the inhaler 3D model turns into the selected color. Upon beginning an inhalation, pressure sensors are triggered and evaluation of the quality of the inhalation is performed by the processing system **270.** The processing system in device calculates and displays a signal corresponding to the quality of the inhalation maneuver at **272.** A visual signal is displayed from the quality LED signal display on the inhalation detecting and monitoring device (jacket) **274,** either a solid red color LED for an incorrect/improper inhalation effort performed at **273** which lets the user know that the inhalation was weak and needs to be harder or stronger in subsequent inhalation in step **276,** or a blinking red LED for an incorrect/improper inhalation effort due to too short duration of the inhalation performed, which indicates to the user that a subsequent inhalation effort should be longer to achieve a proper inhalation at **278** and a solid green LED light display which meets all criteria necessary to deliver a dose of the dry powder to the subject at step **279.**

In a further embodiment for use in conjunction with diabetes treatment, a glucometer may be integrated into a detachable inhalation detecting and monitoring device/jacket in which the jacket can determine glucose levels in a subject upon the measuring of glucose levels with a smart glucose strip and a sample of blood and to minimize the number of apparatus required for use by a subject in the treatment regime. The smart glucose strip can communicate with a wireless transceiver in the inhalation detecting and monitoring apparatus to determine glucose levels prior in order to determine a proper dose required by a patient to lower the patient's blood glucose levels as calculated from the information obtain from the blood sample.

In an alternate embodiment of use and shown in FIG. 28, the steps to train a subject to use the inhaler steps **280-298** are similar or the same as steps **250-274,** however the device is structured and programmed with different communication codes and visual display signals. In this embodiment, the inhalation detecting and monitoring device comprises alternate device visual signals inherent of the device which can be solely be displayed on the device, or linked to a mobile phone or tablet. Upon the inhalation and performing steps **280-296,** in step **297,** the inhalation parameters are evaluated for quality of inhalation, followed by calculating and displaying a signal corresponding to the quality the inhalation in **298** by the processing programs. In step **299,** a graphical interface on a smart phone/tablet displays a graph of an acceptable inhalation. A solid red graphical indicator denotes an incorrect/improper inhalation maneuver/effort and the user is instructed to inhaler harder in a subsequent inhalation **301** until a solid green graphical indicator display is achieve indicating a proper inhalation effort for dosing a dry powder. In step **300,** if the graphical interface indicator displays a red and green graph, the inhalation effort is incorrect/improper, in order to achieve a solid green graphical display, the user is instructed to inhale for a longer period of time. Once the user achieves a solid green graphical interface after several trials, the user is properly train with the inhaler and the detachable detecting and sensing device is powered off and the user can be sent home properly trained to begin it dosing regimen.

In a particular embodiment, the inhaler accessory apparatus is useful for dry powder inhalers, in particular, with a unit dose cartridge, and a drug delivery formulation comprising, for example, diketopiperazine, in particular, fumaryl diketopiperazine and an active ingredient such as peptides and proteins, including, endocrine hormones, including, parathyroid hormone, insulin, oxyntomodulin and glucagon-like peptide 1; symlin or pramlintide acetate, nicotine, neurotransmitters, including cannabinoids, 5-hydroxytryptamine, dopaminergic, prostacyclin, opioid agonists and antagonists. In some embodiments, the active ingredient in the formulations comprises one or more of the active agents, which include, but are not limited to salmeterol, epinephrine, tacrolimus, vancomycin, linezolid, filgastrin, fentanyl, cannabinoids, including, cannabidiols and tetrahydrocannabinol (THC), or derivatives thereof; palonosetron, amphotericin B, phosphodiesterase inhibitors, including, PDE5 inhibitors such as sildenafil, avanafil, vardenafil and tadalafil; prostaglandins, prostacyclin, including treprostinil, neurotransmitter agonists, neurotransmitter antagonists, including anti-nociceptive agents, opioid analgesics such as delta opioid agonists and antagonists, kappa opioid receptor agonists and antagonists, mu opioid receptor agonist and antagonists, nicotine, norepinephrine-dopamine reuptake inhibitor (NDRI) and a nicotinic receptor antagonist, nicotinic acetylcholine receptor agonist, varenicline, cytisine, bupropion, derivatives thereof, pharmaceutically acceptable salts thereof, or combinations thereof.

### Example 1

### Using an Integrated Training Device

A 60 year old Type I diabetic is instructed to receive inhaled insulin for prandial treatment therapy, which is provided from a dry powder inhalation system, because she has an elevated hemoglobin A1c and is considered out of control. The patient uses an insulin pump for basal insulin. The patient is trained for wireless inhalation using a device as illustrated in FIG. 1 with a removable inhalation accessory apparatus as shown in FIGs. 3-5. The patient is given the device and asked to take a deep rapid breath in using the training device which may or may not include the medicament.

Pressure sensors on the inhalation apparatus are used to detect pressure drop during the inhalation and the data is transmitted to a Bluetooth enabled tablet with an associated application. Color detection sensors detect the cartridge (with substance or empty) color and the data is used to identify the threshold region for minimal inhalation pressure. The data is collected on the tablet having a programmed application which can read radio signals from the device and the patient is able to view the data in real-time on a display screen. The patient's first inhalation attempt is too slow and is indicated on-screen as entering a red "unacceptable region" (B region) of FIG.12. The patient is instructed to take another rapid breath in that is slightly faster and deeper than the previous attempt. Upon completion of the inhalation, the graph illustrates that the patient's inhalation maneuver was acceptable and entirely in the acceptable region of the graph (C region) of FIG. 12. Upon being comfortable with the training, the patient is clear for use of a similar inhaler device with the medicament loaded therein.

The patient is prescribed a dry powder inhaler similar to the type that illustrated in FIG. 1 and cartridges filled with an inhalable insulin of various doses for treatment of the patient's diabetes. Six months after prescribing the inhaled insulin, the patient's diabetes is diagnosed as under control.

### Example 2

**Using an Attachable/Detachable Training Device:** A 59 year old Type II diabetic is instructed to receive inhaled insulin from a dry powder inhalation system. The patient has requested the inhalation system for convenient reasons. The patient is trained for wireless inhalation using a device as illustrated in FIG. 1. The patient is given the device of FIG. 1 equipped with an attachable inhaler apparatus similar to that of FIG. 3-5 and asked to take a deep rapid breath in using the training device.

Examples of inhalation maneuvers performed by a subject are illustrated in FIGs. 23, 24, 25 and 26 and described above.

The pressure and color identification data is collected on a mobile phone and the patient is able to view the data in real-time on a display screen. The patient's first attempt is acceptable as indicated by the threshold vs. inhalation data graphed or otherwise visually indicated as a result of the software. Upon being comfortable with the training, the patient is clear for use of the device.

The patient attachable sensor is removed from the dry powder inhaler. The patient is given the dry powder inhaler and cartridges filled with inhalable insulin for treatment of the patient's diabetes. Six months after prescribing the inhaled insulin, the patient's diabetes is diagnosed as under control and the patient comments on the great convenience of the device.

The preceding disclosures are illustrative embodiments. It should be appreciated by those of skill in the art that the techniques disclosed herein elucidate representative techniques that function well in the practice of the present disclosure. However, those of skill in the art should, in light of the present disclosure, appreciate that many changes can be made in the specific embodiments that are disclosed and still obtain a like or similar result without departing from the scope of this disclosure.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the disclosed embodiments are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the disclosed embodiments (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the disclosed embodiments and does not pose a limitation on the scope of the embodiments otherwise claimed. No language in the specification should be construed as indicating any non-claimed element essential to the practice of disclosed and contemplated embodiments.

Specific embodiments disclosed herein may be further limited in the claims using consisting of or and consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments so claimed are inherently or expressly described and enabled herein.

Groupings of alternative elements or embodiments disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments are described herein, including the best mode known to the authors of this disclosure for carrying the disclosed and contemplated embodiments. Of course, variations on these described embodiments will become apparent to those of ordinary skill in the art upon reading the foregoing description. The authors expect skilled artisans to employ such variations as appropriate, and the authors intend for the disclosed and contemplated embodiments to be practiced otherwise than specifically described herein. it is to be understood that the embodiments disclosed and contemplated herein are illustrative of the principles of the conceived invention. Other modifications that may be employed are within the scope of this disclosure. Thus, by way of example, but not of limitation, alternative configurations may be utilized in accordance with the teachings herein. Accordingly, the disclosed embodiments are not limited to that precisely as shown and described.

## Claims

1. An inhaler accessory apparatus (220) comprising:
a body (224) configured for mounting onto or connection with an inhaler;
a microprocessor (32),
at least two pressure sensors (191,192) comprising
a first pressure sensor (192), distal to an inhaler medicament/airflow delivery conduit of said dry powder inhaler, configured to obtain an absolute pressure from the environment around said dry powder inhaler; and
a second pressure sensor (191), proximal to said inhaler medicament/airflow delivery conduit (16), configured to obtain a differential pressure generated from said inhaler medicament/airflow delivery conduit when in use, and
a pressure equalization channel (234) between said first pressure sensor and said second pressure sensor through which said first pressure sensor and said second pressure sensor pneumatically communicate during use, wherein said first pressure sensor and said second pressure sensor are configured to generate at least one signal each, the microprocessor being configured to process said signals to produce customized output adapted to the atmospheric conditions, and the microprocessor being further configured to generate a pressure differential versus time curve on a display concurrently with or immediately after a user's inhalation; optionally, wherein said first pressure sensor and said second pressure sensor are digital sensors and their output is in digital form.

2. The inhaler accessory apparatus (220) of claim 1, wherein said second pressure sensor is an analog sensor and its output is in analog form and wherein the apparatus further comprises additional circuitry to condition, filter, amplify and/or convert a sensor signal to digital form.

3. The inhaler accessory apparatus (220) of claim 1, wherein said second pressure sensor is configured to detect a pressure drop measured in said inhaler medicament/airflow delivery conduit.

4. The inhaler accessory apparatus (220) of claim 1, wherein said second pressure sensor is configured to detect atmospheric pressure; optionally further comprising a photo sensor which is configured to detect visible light or augmented light signals emitted from said inhaler in use in ambient conditions; and further optionally, further comprising a light emitting diode.

5. The inhaler accessory apparatus (220) of claim 1, further comprising a laser beam, a Doppler sensor, infrared sensor or other sensing beam configured to detect characteristic profile of parts of the inhaler, inhaler cartridge, or other medicament package; or further comprising an accelerometer and wherein the inhaler comprises a dry powder inhaler; optionally wherein said dry powder inhaler comprises a cartridge and a dry powder formulation.

6. The inhaler accessory apparatus (220) of claim 5, wherein the dry powder formulation comprises a diketopiperazine and at least one active ingredient.

7. The inhaler accessory apparatus (220) of claim 1, wherein said second pressure sensor is configured to detect a pressure drop measured in said inhaler and the first pressure sensor is configured to be used in conjunction with the second pressure sensor to adjust, if necessary, for atmospheric conditions before identifying the pressure drop.

8. The inhaler accessory apparatus (220) of claim 1, further comprising one or more of: a color detection sensor for sensing color of an inhaler cartridge or other medicament package loaded into the inhaler, an RFID reader for reading an RFID tag in a cartridge or other medicament package loaded in the inhaler, and image detection sensors capable of identifying characters, codes or textual information provided on the inhaler or the inhaler cartridge or other medicament package.

9. The inhaler accessory apparatus (220) of claim 1, further comprising a wireless transceiver for transmitting data received from the at least two sensors to a remote processing system.

10. The inhaler accessory apparatus (220) of claim 9, further comprising an electronic board and wherein the microprocessor, sensors and wireless transceiver are connected to and/or disposed on the electronic board.

11. The inhaler accessory apparatus (220) of claim 1, further comprising visual indicators or a display for showing differences between an idealized or predetermined inhalation parameters and the attained/performed inhalation maneuver of a user; optionally wherein the visual indicators are a pass/fail, or green/red indicators to a user.

12. The inhaler accessory apparatus of claim 6, wherein the at least one active ingredient comprises insulin or an insulin analog, sildenafil, avanafil, vardenafil, treprostinil, tadalafil, epinephrine, tetrahydrocannabinol and cannabidiol.

## Patentansprüche

1. Inhalator-Zubehörgerät (220), umfassend:
einen Körper (224), der zum Anbringen an oder Verbinden mit einem Inhalator ausgelegt ist;
einen Mikroprozessor (32), mindestens zwei Drucksensoren (191, 192), umfassend einen ersten Drucksensor (192), distal zu einer Inhalatormedikament-/Luftstromabgabeleitung des Trockenpulverinhalators, der dazu ausgelegt ist, einen Absolutdruck aus der Umgebung um den Trockenpulverinhalator zu erhalten; und
einen zweiten Drucksensor (191), proximal zu der Inhalatormedikament-/Luftstromabgabeleitung (16), der dazu ausgelegt ist, einen Differenzdruck zu erhalten, der von der Inhalatormedikament-/Luftstromabgabeleitung erzeugt wird, wenn er in Gebrauch ist, und
einen Druckausgleichskanal (234) zwischen dem ersten Drucksensor und dem zweiten Drucksensor, durch den der erste Drucksensor und der zweite Drucksensor während des Gebrauchs pneumatisch kommunizieren,
wobei der erste Drucksensor und der zweite Drucksensor dazu ausgelegt sind, jeweils mindestens ein Signal zu erzeugen, wobei der Mikroprozessor dazu ausgelegt ist, die Signale zu verarbeiten, um eine individualisierte Ausgabe zu produzieren, die an die atmosphärischen Bedingungen angepasst ist, und wobei der Mikroprozessor ferner dazu ausgelegt ist, eine Druckdifferenz-Zeit-Kurve auf einer Anzeige gleichzeitig mit oder unmittelbar nach der Inhalation eines Benutzers zu erzeugen;
wobei gegebenenfalls der erste Drucksensor und der zweite Drucksensor digitale Sensoren sind und ihre Ausgabe in digitaler Form vorliegt.

2. Inhalator-Zubehörgerät (220) nach Anspruch 1, wobei der zweite Drucksensor ein analoger Sensor ist und seine Ausgabe in analoger Form vorliegt, und wobei das Gerät ferner eine zusätzliche Schaltungsanordnung zum Konditionieren, Filtern, Verstärken und/oder Umwandeln eines Sensorsignals in digitale Form umfasst.

3. Inhalator-Zubehörgerät (220) nach Anspruch 1, wobei der zweite Drucksensor dazu ausgelegt ist, einen Druckabfall zu detektieren, der in der Inhalatormedikament-/Luftstromabgabeleitung gemessen wird.

4. Inhalator-Zubehörgerät (220) nach Anspruch 1, wobei der zweite Drucksensor dazu ausgelegt ist, Atmosphärendruck zu detektieren; gegebenenfalls weiter umfassend einen Fotosensor, der dazu ausgelegt ist, sichtbares Licht oder erweiterte Lichtsignale, die von dem Inhalator bei Gebrauch emittiert werden, unter Umgebungsbedingungen zu detektieren; und ferner gegebenenfalls umfassend eine Leuchtdiode.

5. Inhalator-Zubehörgerät (220) nach Anspruch 1, ferner umfassend einen Laserstrahl, einen Doppler-Sensor, Infrarotsensor oder anderen Abfühlstrahl, der dazu ausgelegt ist, ein charakteristisches Profil von Teilen des Inhalators, der Inhalatorpatrone oder einer anderen Medikamentenpackung zu detektieren; oder ferner umfassend einen Beschleunigungsmesser, und wobei der Inhalator einen Trockenpulverinhalator umfasst; wobei der Trockenpulverinhalator gegebenenfalls eine Patrone und eine Trockenpulverformulierung umfasst.

6. Inhalator-Zubehörgerät (220) nach Anspruch 5, wobei die Trockenpulverformulierung ein Diketopiperazin und mindestens einen Wirkstoff umfasst.

7. Inhalator-Zubehörgerät (220) nach Anspruch 1, wobei der zweite Drucksensor dazu ausgelegt ist, einen in dem Inhalator gemessenen Druckabfall zu detektieren, und der erste Drucksensor dazu ausgelegt ist, in Verbindung mit dem zweiten Drucksensor verwendet zu werden, um, falls erforderlich, atmosphärische Bedingungen anzupassen, bevor der Druckabfall identifiziert wird.

8. Inhalator-Zubehörgerät (220) nach Anspruch 1, ferner umfassend eines oder mehrere von: einem Farbdetektionssensor zum Abfühlen der Farbe einer Inhalatorpatrone oder anderen Medikamentenpackung, die in den Inhalator geladen ist, einem RFID-Leser zum Lesen eines RFID-Tags in einer Patrone oder anderen Medikamentenpackung, die in dem Inhalator geladen ist, und Bilddetektionssensoren, die in der Lage sind, Zeichen, Codes oder Textinformationen zu identifizieren, die auf dem Inhalator oder der Inhalatorpatrone oder anderen Medikamentenpackung bereitgestellt sind.

9. Inhalator-Zubehörgerät (220) nach Anspruch 1, ferner umfassend einen drahtlosen Sendeempfänger zum Übertragen von Daten, die von den mindestens zwei Sensoren empfangen werden, an ein Fernverarbeitungssystem.

10. Inhalator-Zubehörgerät (220) nach Anspruch 9, ferner umfassend eine Elektronikplatine, und wobei der Mikroprozessor, die Sensoren und der drahtlose Sendeempfänger mit der Elektronikplatine verbunden und/oder darauf angeordnet sind.

11. Inhalator-Zubehörgerät (220) nach Anspruch 1, ferner umfassend visuelle Indikatoren oder eine Anzeige zum Zeigen von Unterschieden zwischen einem idealisierten oder vorbestimmten Inhalationsparameter und dem erreichten/durchgeführten Inhalationsmanöver eines Benutzers; wobei die visuellen Indikatoren gegebenenfalls ein Bestanden/Fehlgeschlagen oder grün/rote Indikatoren für einen Benutzer sind.

12. Inhalator-Zubehörgerät nach Anspruch 6, wobei der mindestens eine Wirkstoff Insulin oder ein Insulinanalogon, Sildenafil, Avanafil, Vardenafil, Treprostinil, Tadalafil, Epinephrin, Tetrahydrocannabinol und Cannabidiol umfasst.

## Revendications

1. Appareil accessoire pour inhalateur (220) comprenant :
un corps (224) configuré pour être monté sur ou connecté à un inhalateur ;
un microprocesseur (32), au moins deux capteurs de pression (191, 192) comprenant un premier capteur de pression (192), distal par rapport à un conduit d'administration de médicament/flux d'air d'inhalateur dudit inhalateur de poudre sèche, configuré pour obtenir une pression absolue de l'environnement autour dudit inhalateur de poudre sèche ; et
un second capteur de pression (191), proximal par rapport audit conduit (16) d'administration de médicament/flux d'air d'inhalateur, configuré pour obtenir une pression différentielle générée depuis ledit conduit d'administration de médicament/flux d'air d'inhalateur lorsqu'il est utilisé, et
un canal d'égalisation de pression (234) entre ledit premier capteur de pression et ledit second capteur de pression par le biais duquel ledit premier capteur de pression et ledit second capteur de pression communiquent pneumatiquement en cours d'utilisation,
dans lequel ledit premier capteur de pression et ledit second capteur de pression sont configurés pour générer au moins un signal chacun, le microprocesseur étant configuré pour traiter lesdits signaux pour produire une sortie personnalisée adaptée aux conditions atmosphériques, et le microprocesseur étant en outre configuré pour générer une courbe de différentiel de pression en fonction du temps sur un afficheur simultanément ou immédiatement après l'inhalation d'un utilisateur ;
éventuellement, dans lequel ledit premier capteur de pression et ledit second capteur de pression sont des capteurs numériques et leur sortie est numérique.

2. Appareil accessoire d'inhalateur (220) selon la revendication 1, dans lequel ledit second capteur de pression est un capteur analogique et sa sortie est sous forme analogique et dans lequel l'appareil comprend en outre des circuits supplémentaires pour conditionner, filtrer, amplifier et/ou convertir un signal de capteur en forme numérique.

3. Appareil accessoire d'inhalateur (220) selon la revendication 1, dans lequel ledit second capteur de pression est configuré pour détecter une chute de pression mesurée dans ledit conduit d'administration de médicament/flux d'air d'inhalateur.

4. Appareil accessoire d'inhalateur (220) selon la revendication 1, dans lequel ledit second capteur de pression est configuré pour détecter la pression atmosphérique ; éventuellement comprenant en outre un photocapteur qui est configuré pour détecter la lumière visible ou des signaux lumineux augmentés émis par ledit inhalateur en cours d'utilisation dans des conditions ambiantes ; et encore éventuellement, comprenant en outre une diode électroluminescente.

5. Appareil accessoire d'inhalateur (220) selon la revendication 1, comprenant en outre un faisceau laser, un capteur Doppler, un capteur infrarouge ou un autre faisceau de détection configuré pour détecter le profil caractéristique de parties de l'inhalateur, de la cartouche d'inhalateur ou d'un autre emballage de médicament ; ou comprenant en outre un accéléromètre et dans lequel l'inhalateur comprend un inhalateur de poudre sèche ; éventuellement dans lequel ledit inhalateur de poudre sèche comprend une cartouche et une formulation de poudre sèche.

6. Appareil accessoire pour inhalateur (220) selon la revendication 5, dans lequel la formulation de poudre sèche comprend une dicétopipérazine et au moins un principe actif.

7. Appareil accessoire d'inhalateur (220) selon la revendication 1, dans lequel ledit second capteur de pression est configuré pour détecter une chute de pression mesurée dans ledit inhalateur et le premier capteur de pression est configuré pour être utilisé conjointement avec le second capteur de pression pour s'ajuster, si nécessaire, aux conditions atmosphériques avant d'identifier la chute de pression.

8. Appareil accessoire d'inhalateur (220) selon la revendication 1, comprenant en outre un ou plusieurs parmi : un capteur de détection de couleur pour détecter la couleur d'une cartouche d'inhalateur ou d'un autre emballage de médicament chargé dans l'inhalateur, un lecteur RFID pour lire une étiquette RFID dans une cartouche ou un autre emballage de médicament chargé dans l'inhalateur, et des capteurs de détection d'image susceptibles d'identifier des caractères, des codes ou des informations textuelles fournis sur l'inhalateur ou sur la cartouche d'inhalateur ou un autre emballage de médicament.

9. Appareil accessoire d'inhalateur (220) selon la revendication 1, comprenant en outre un émetteur-récepteur sans fil pour transmettre des données reçues des au moins deux capteurs à un système de traitement à distance.

10. Appareil accessoire d'inhalateur (220) selon la revendication 9, comprenant en outre une carte électronique et dans lequel le microprocesseur, les capteurs et l'émetteur-récepteur sans fil sont connectés à et/ou disposés sur la carte électronique.

11. Appareil accessoire d'inhalateur (220) selon la revendication 1, comprenant en outre des témoins visuels ou un afficheur pour montrer des différences entre des paramètres d'inhalation idéalisés ou prédéterminés et la manœuvre d'inhalation atteinte/exécutée d'un utilisateur ; éventuellement dans lequel les témoins visuels sont des témoins de réussite/échec ou vert/rouge pour un utilisateur.

12. Appareil accessoire pour inhalateur selon la revendication 6, dans lequel l'au moins un principe actif comprend de l'insuline ou un analogue d'insuline, du sildénafil, de l'avanafil, du vardénafil, du tréprostinil, du tadalafil, de l'épinéphrine, du tétrahydrocannabinol et du cannabidiol.
